# FASCICULE DE BREVET EUROPEEN

(11) **EP 0 514 269 B1**
(45) Date de publication et mention de la délivrance du brevet: **02.11.1995**
(21) Numéro de dépôt: 92401320.4
(22) Date de dépôt: 14.05.1992
(51) Int. Cl.: C07C 257/08, C07C 251/24, C07C 323/45, C07C 257/18, A61K 31/19, A61K 7/06, A61K 7/40

(54) **Nouveaux composés aromatiques dérivés d'imine, leur procédé de préparation et leur utilisation en médecine humaine et vétérinaire ainsi qu'en cosmétique**
Iminoderivate, Verfahren zu ihrer Herstellung und ihre Verwendung in der Human- und Tiermedizin und in der Kosmetik
Imino-derivatives, process for their preparation and their use in human and veterinary medicine and in cosmetics

(30) Priorité: 15.05.1991 FR 9105883
(43) Date de publication de la demande: 19.11.1992
(73) Titulaire: CENTRE INTERNATIONAL DE RECHERCHES DERMATOLOGIQUES GALDERMA, ( CIRD GALDERMA), 06560 Valbonne (FR)
(72) Inventeur: Rocher, Jean-Philippe, F-74240 Gaillard (FR); Bernardon, Jean-Michel, F-06650 - Nice (FR)
(74) Mandataire: Stalla-Bourdillon, Bernard

(56) Documents cités:
- EP-A- 0 232 199
- US-A- 3 669 974
- US-A- 4 264 505
- CHEMICAL ABSTRACTS, vol. 92, no. 9, 3 mars 1980, Columbus, Ohio, US; abstract no. 76036M, "Cumylphenol derivatives. III. Synthesis and electronic spectra of some cumylphenol-derived arylazomethines", page 636
- CHEMICAL ABSTRACTS, vol. 77, no. 17, 23 octobre 1972, Columbus, Ohio, US; abstract no. 113925, "Screened phenols. VI. Azomethines, derivatives of 3,5-di-tert-butyl-4-hydroxybenzaldehyde", page 409
- CHEMICAL ABSTRACTS, vol. 111, no. 7, 14 août 1989, Columbus, Ohio, US; abstract no. 56768J, "Synthesis of sterically hindered diarylamidine phenols and properties of radicals formed from them", page 674

## Description

La présente invention a pour objet de nouveaux composés aromatiques, leur procédé de préparation et leur utilisation en médecine humaine et vétérinaire et en cosmétique.

Ces nouveaux composés trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité anti-tumorale. En outre, ces composés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies.

L'état de la technique relatif au traitement des cornéopathies est essentiellement constitué par EP-A-232 199 qui décrit l'utilisation de dérivés benzamido arômatiques.

Les composés selon l'invention peuvent être représentés par la formule générale suivante : 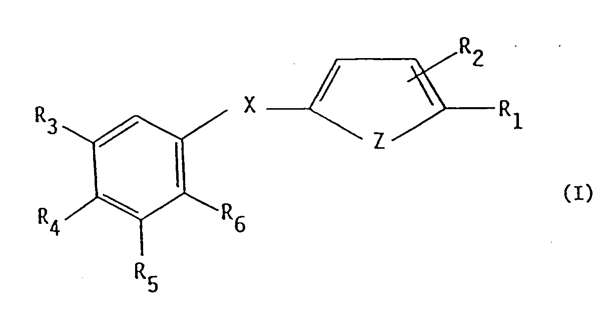
dans laquelle :
R₁ représente un atome d'hydrogène, le groupe OH, le radical -CH₃, le radical -CH₂OH, le radical -COR₇, le radical -CH(OH)CH₃, le radical -CH₂OCOR₈, le radical -SO₂R₉, le radical SOR₉ ou le radical -SR₉,
R₇ représentant un atome d'hydrogène, le groupe OH, le radical -OR₁₀, le radical : 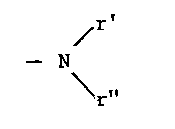
un radical alkyle inférieur, un radical monohydroxyalkyle, un radical polyhydroxyalkyle ou le reste d'un sucre,
R₈ représentant un radical alkyle linéaire ou ramifié, ayant de 1 à 20 atomes de carbone, un radical alkényle ayant de 2 à 20 atomes de carbone ou le reste d'un sucre,
R₉ représentant le groupe OH, un radical alkyle inférieur ou le radical 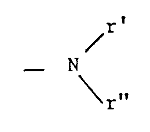
R₁₀ représentant un radical alkyle ayant de 1 à 20 atomes de carbone ou un radical alkényle ayant de 2 à 20 atomes de carbone,
r' et r'', identiques ou différents, représentent un atome d'hydrogène, un radical alkyle inférieur, un radical aryle, un radical aralkyle, un reste d'aminoacide, un reste de sucre, un reste de sucre aminé ou un hétérocyle ou r' et r'' pris ensemble forment un hétérocycle,
R₂ et R₆ représentent un atome d'hydrogène, le groupe OH, un radical alkyle inférieur, un radical alkoxy ayant de 1 à 6 atomes de carbone, un atome de fluor, un atome de chlore ou le groupe CF₃,
R₃ et R₅ représentent un radical alkyle α,α'-disubstitué ayant de 4 à 12 atomes de carbone ou un radical cycloalkyle mono ou polycyclique ayant de 5 à 12 atomes de carbone dont le carbone de liaison est trisubstitué,
R₄ représente un atome d'hydrogène, le groupe OH, un radical alkoxy ayant de 1 à 6 atomes de carbone, un radical alkyle α,α'-disubstitué ayant de 4 à 12 atomes de carbone,
R₃ et R₄ ou R₄ et R₅ pris ensemble peuvent former, avec le noyau benzénique adjacent, un cycle à 5 ou 6 atomes de carbone substitués par 2 à 6 groupes méthyles,
Z représente le radical divalent -CH=CR₁₁-
R₁₁ représentant un atome d'hydrogène, le groupe OH ou un radical alkyle inférieur,
X est choisi parmi les radicaux :
(i) - CR₁₃= N -
(ii) - N = CR₁₃-
(iii) 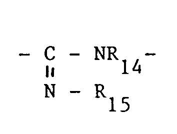
(iv) 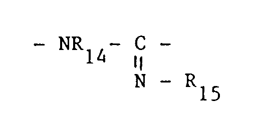
R₁₃ représentant le radical R₁₆, le radical OR₁₆, le radical -SR₁₆ ou le radical : 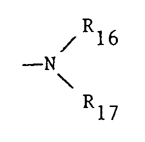
R₁₆ et R₁₇ représentant un atome d'hydrogène, un radical alkyle inférieur, un radical fluoroalkyle inférieur, un radical alkényle ayant de 2 à 6 atomes de carbone, un radical alkynyle ayant de 2 à 6 atomes de carbone, un radical aryle ou un radical aralkyle,
R₁₄ représentant un radical alkyle inférieur,
R₁₅ représentant un radical alkyle inférieur ou un radical fluoroalkyle inférieur,
à l'exclusion des composés de Formule (I) dans laquelle R₃ et R₅ sont identiques lorsque X représente le radical - CR₁₃= N - , R₁₃ représentant un atome d'hydrogène.

Cette exclusion vise essentiellement les composés décrits par Bruck et al., Zh. obshch. Khim (1972) (42) 7, p.1603-8 (C.A. Vol.77, 113 922K, 1972).

La présente invention a également pour objet les sels de ces composés formés aussi bien avec des bases qu'avec des acides et les isomères optiques desdits composés de formule (I).

Lorsque les composés selon l'invention se présentent sous forme de sels, par addition d'une base, il s'agit alors de sels d'un métal alcalin ou alcalino-terreux ou encore de zinc ou d'une amine organique.

Lorsque les composés se présentent sous forme de sels, par addition d'un acide, il s'agit de sels pharmaceutiquement ou cosmétiquement acceptable obtenus par addition d'un acide minéral ou organique en particulier l'acide chlorhydrique, sulfurique, acétique, citrique, fumarique, hémisuccinique, maléique et mandélique.

Par radical alkyle inférieur, on entend un radical ayant de 1 à 6 atomes de carbone et de préférence les radicaux méthyle, éthyle, isopropyle, butyle et tertiobutyle.

Par radical alkoxy ayant de 1 à 6 atomes de carbone, on doit entendre notamment un radical méthoxy, éthoxy, isopropoxy ou butoxy.

Par radical alkyle α,α′-disubstitué ayant de 4 à 12 atomes de carbone, on doit notamment entendre un radical tert-butyle, 1,1-diméthyl propyle, 1-méthyl 1-éthyl propyle, 1-méthyl 1-éthyl hexyle ou 1,1-diméthyl décyle.

Par radical cycloalkyle mono ou polycyclique ayant de 5 à 12 atomes de carbone dont le carbone de liaison est trisubstitué, on doit entendre le radical 1-méthyl cyclohexyle ou 1-adamantyle.

Par radical monobydroxyalkyle, on doit entendre un radical ayant de 1 à 6 atomes de carbone, notamment un radical 2-hydroxyéthyle, 2-hydroxypropyle ou 3-hydroxypropyle.

Par radical polyhydroxyalkyle, on doit entendre un radical contenant de 2 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on doit entendre un radical phényle éventuellement substitué par au moins un atome d'halogène, un hydroxyle ou une fonction nitro.

Par radical aralkyle, on doit entendre le radical benzyle ou phénéthyle éventuellement substitué par au moins un atome d'halogène, un hydroxyle ou une fonction nitro.

Par radical alkényle ayant de 2 à 6 atomes de carbone on doit entendre notamment le radial vinyle, propényle, 2-méthyl-propényle ou butène-2-yle.

Par radical alkynyle ayant de 2 à 6 atomes de carbone, on doit entendre notamment le radical propargyle.

Par radical fluoroalkyle inférieur on doit entendre un radical ayant de 1 à 6 atomes de carbone et de 3 à 7 atomes de fluor tels que -CF₃ et -C₂F₅.

Lorsque R₈ ou R₁₀ représente un radical alkyle ayant de 1 à 20 atomes de carbone ou un radical alkényle ayant de 2 à 20 atomes de carbone on doit entendre des radicaux linéaires ou ramifiés éventuellement substitués par un ou plusieurs groupes hydroxyles ou un ou plusieurs atomes de fluor.

Par reste d'aminoacide on doit entendre un reste dérivant par exemple de l'un des 20 aminoacides de configuration L ou D (ou leur mélange racémique) constitutifs de protéines de mammifères.

Par reste d'un sucre on doit entendre un reste dérivant par exemple de glucose, galactose ou mannose.

Par reste d'un sucre aminé, on doit entendre un reste dérivant par exemple de glucosamine, de galactosamine ou de mannosamine.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus, on peut notamment citer les suivants :
1) Acide 4-(N-méthyl-5,6,7,8-tétrahydro-5,5,8,8-tétraméthyl-2-naphtylcarboxamidino) benzoïque,
2) Acide 4-(5,6,7,8-tétrahydro-5,5,8,8-tétraméthyl-2-naphtylcarboxamidino) benzoïque,
3) Acide 4-[5-(1-adamantyl)-2 hydroxy-4-méthoxybenzylidèneamino] benzoïque,
4) Acide 4-[3-(1-adamantyl)-4-méthoxybenzamidino]benzoïque,
5) Chlorhydrate de l'acide 4-[N-méthyl-3-(1-adamantyl)-4-méthoxybenzamidino] benzoïque,
6) Acide 4-[3-(1-adamantyl)-4-méthoxyphénylformamidinométhyl thioéther] benzoïque,
7) Acide 4-[3-(1-adamantyl)-4-méthoxyphénylformamidinométhyléther] benzoïque,
8) 4-[3-(1-adamantyl)-4-méthoxybenzylidèneamino] benzoate de méthyle,
9) Acide 4-[3-(1-adamantyl)-4-méthoxybenzylidèneamino] benzoïque,
10) Acide 4-(3-tert-butyl-4-méthoxybenzamidino)benzoïque
11) 4-(5,6,7,8-tétrahydro-5,5,8,8-tétraméthyl-2- naphtylcarboxamidino)benzoate de méthyle
12) Alcool 4-(5,6,7,8-tétrahydro-5,5,8,8-tétraméthyl-2- naphtylcarboxamidino)benzylique
13) 4-(5,6,7,8-tétrahydro-5,5,8,8-tétraméthyl-2- naphtylcarboxamidino) toluène
14) 4-(5,6,7,8-tétrahydro-5,5,8,8-tétraméthyl-2- naphtylcarboxamidino) benzamide
15) Acide 2-hydroxy-4-(5,6,7,8-tétrahydro-5,5,8,8-tétraméthyl-2-naphtylcarboxamidino) benzoïque
16) 4-(α-chloro-5,6,7,8-tétrahydro-5,5,8,8-tétraméthyl-2- naphtylméthylimino)benzoate d'allyle
17) Acide 4-(N¹-phényl-5,6,7,8-tétrahydro-5,5,8,8-tétraméthyl-2-naphtylcarboxamidino) benzoïque
18) Chlorhydrate de l'acide 4-(N¹-phényl-5,6,7,8-tétrahydro-5,5,8,8-tétraméthyl-2-naphtylcarboxamidino) benzoïque
19) Acide 4-(N¹-benzyl-5,6,7,8-tétrahydro-5,5,8,8-tétraméthyl-2-naphtyl-carboxamidino)benzoïque
20) Acide 4-(N¹,N¹-diméthyl-5,6,7,8-tétrahydro-5,5,8,8-tétraméthyl-2-naphtyl-carboxamidino)benzoïque
21) Acide 4-[N²-(5,6,7,8-tétrahydro-5,5,8,8-tétraméthyl-2-naphtyl)amidino]benzoïque
22) Acide 4-[N¹-phényl-N2-(5,6,7,8-tétrahydro-5,5,8,8-tétraméthyl-2-naphtyl)amidino] benzoïque
23) Chlorhydrate du 4-(5,6,7,8-tétrahydro-5,5,8,8-tétraméthyl-2-naphtylcarboxamidino) phénol
24) Acide 4-(N¹-méthyl-N²-méthyl-5,6,7,8-tétrahydro-5,5,8,8-tétraméthyl-2-naphtylcarboxamidino)benzoïque
25) Acide 4-[N-(3,3,3-trifluoroéthyl)-5,6,7,8-tétrahydro-5,5,8,8-tétraméthyl-2-naphtylcarboxamidino] benzoïque.

La présente invention a également pour objet à titre de composés intermédiaires les composés de formule : 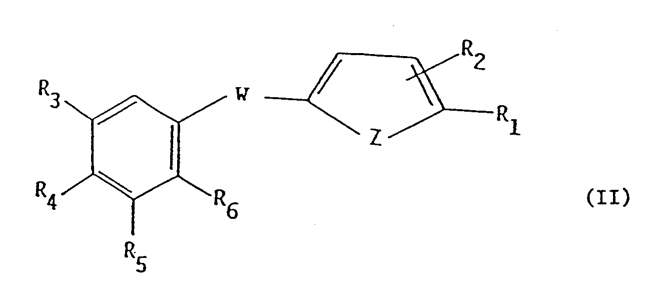
dans laquelle :
W représente un radical choisi parmi : 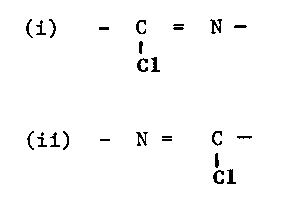 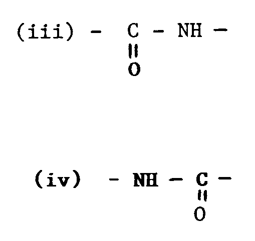
Z et R₂ à R₆ ayant les mêmes significations données ci-dessus pour la formule (I)
R₁ a les mêmes significations données ci-dessus pour la formule (I) lorsque W est soit le radical (i) soit le radical (ii), ou R₁ représente le radical -COOCH₂-CH=CH₂ lorsque W est soit le radical (iii) soit le radical (iv).

La présente invention a également pour objet le procédé de préparation des composés de formule (I).

Lorsque X représente une liaison imine avec X = (i) les composés sont obtenus par réaction d'un benzaldéhyde substitué avec un para-amino benzoate d'allyle éventuellement substitué dans un solvant anhydre tel le chlorure de méthylène en présence d'un déshydratant par exemple l'alumine basique.

Lorsque X représente une liaison imine avec X = (ii) les composés sont obtenus par réaction d'une aniline substituée sur un aldéhyde aromatique substitué par une fonction acide qui est protégée sous forme d'ester allylique dans les mêmes conditions que précédemment.

Lorsque X représente une liaison imidate, thioimidate ou amidine avec X = (i) les composés selon l'invention sont préparés d'après le schéma réactionnel suivant : 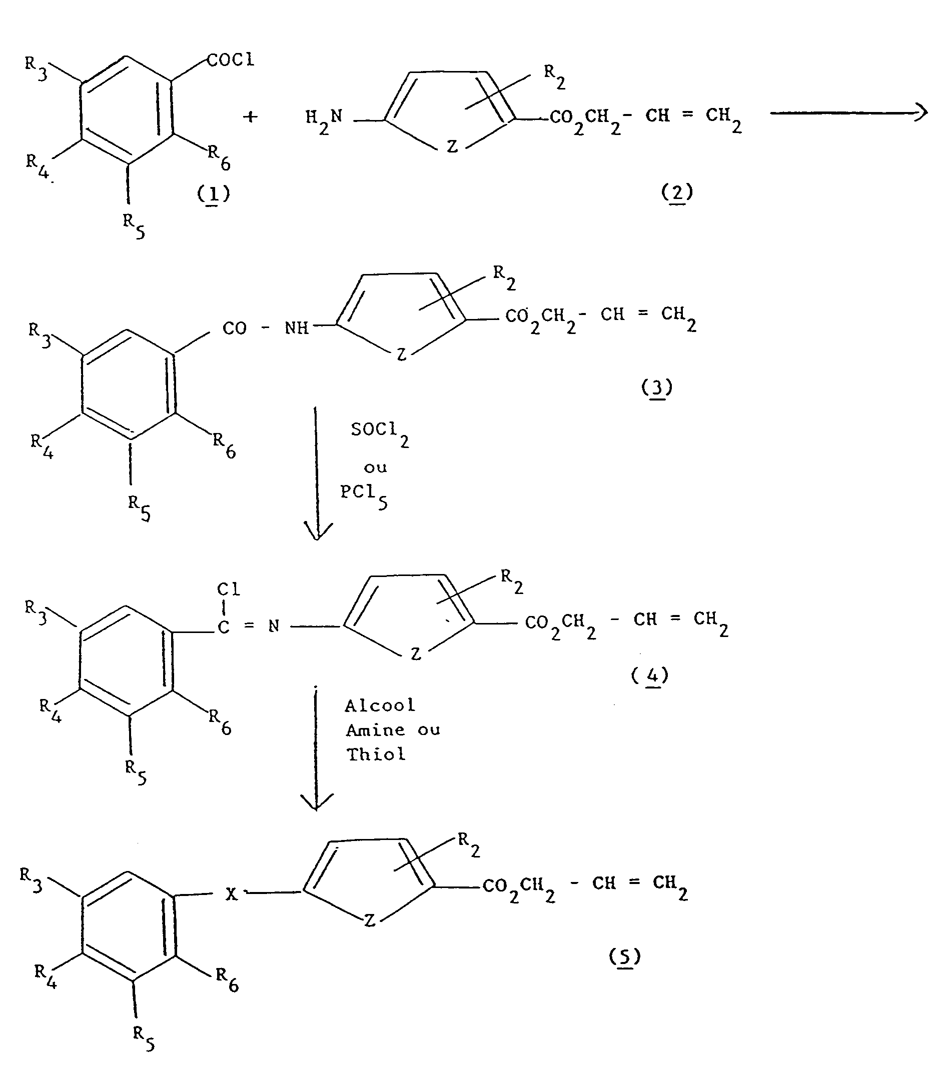
La première étape consiste à faire réagir en milieu anhydre, dans un solvant organique tel que le tétrahydrofuranne ou le chlorure de méthylène contenant une amine tertiaire (pyridine ou triéthylamine) une forme activée d'un acide benzoïque substitué, par exemple un chlorure d'acide (1) ou un anhydride mixte sur un para-amino benzoate d'allyle éventuellement substitué (2) . La réaction est conduite à température ambiante et sous agitation.

L'amide (3) ainsi obtenu est converti en iminochlorure (4) par action du chlorure de thionyle, du pentachlorure de phosphore ou du phosgène.

Par réaction du composé (4) avec une amine, un alcool ou un thiol en présence d'une amine tertiaire et d'un hydrure alcalin dans un solvant organique tel que le tétrahydrofuranne ou le chlorure de méthylène, on obtient le composé de formule (5).

Lorsque X représente une liaison imidate, thioimidate, ou amidine avec X = (ii) la préparation est effectuée de la même manière que précédemment en partant des composés (6) et (7) suivants : 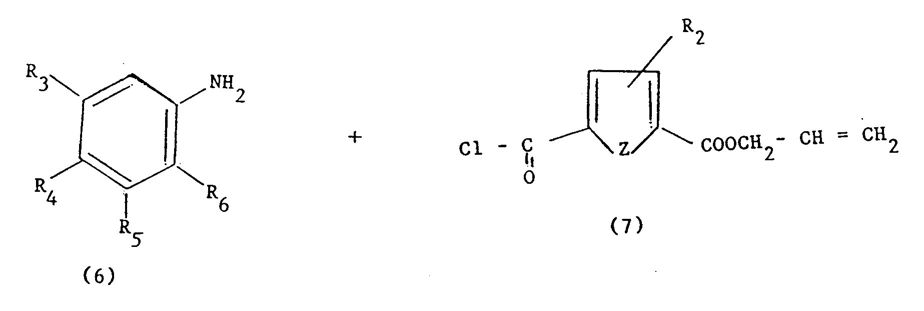
Le passage de l'ester à l'acide libre peut être effectué dans les 4 cas ci-dessus au moyen d'un catalyseur, tel que certains complexes de métaux de transition par exemple le tetrakis triphényl phosphine palladium (0) en présence d'une amine secondaire ou du sel de sodium du malonate de diéthyle ou en milieu alcalin de préférence en présence d'une solution de soude méthanolique.

Lorsque X représente une liaison amidine correspondant aux formules (iii) et (iv) la synthèse est réalisée selon le procédé classique de PINNER en condensant une aniline substituée sur un nitrile aromatique.

La présente invention a également pour objet à titre de médicament les composés de formule (I) telle que définie ci-dessus.

Les composés selon l'invention présentent une bonne stabilité à la lumière et à l'oxygène.

Ces composés présentent une activité dans le test de différenciation des cellules (F9) de tératocarcinome embryonnaire de souris (Cancer Research 43, p. 5268, 1983)et/ou dans le test d'inhibition de l'ornithine décarboxylase après induction par le TPA chez la souris (Cancer Research 38, p.793-801, 1978). Ces tests montrent les activités des composés respectivement dans les domaines de la différenciation et de la prolifération.

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, les acnés nodulokystiques, conglobata, les acnés séniles, les acnés secondaires telles que l'acné solaire, médicamenteuse, professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux.
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma, ou l'atopie respiratoire ou l'hypertrophie gingivale ; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient d'origine virale telles que verrues vulgaires, les verrues planes et l'épidermodysplasie verruciforme, les papillomaroses orales florides, les proliférations pouvant également être induites par les ultra-violets notamment dans le cas des épithélioma baso et spino cellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photoinduitou chronologique ou à réduire les pigmentations et les kératoses actiniques.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou réparer les troubles de la cicatrisation ou les vergétures.
10) Pour lutter contre les troubles de la fonction sébacée tels que l'hyper séborrhée de l'acné ou la séborrhée simple.
11) Dans le traitement de situations cancéreuses ou précancéreuses en particulier au niveau cutané.
12) Dans le traitement d'affections inflammatoires telles que l'arthrite.

La présente invention a également pour objet des compositions médicamenteuses contenant au moins un composé de formule (I) telle que définie ci-dessus, ou un de ses sels.

La présente invention a donc aussi pour objet une nouvelle composition médicamenteuse destinée notamment au traitement des affections susmentionnées, caractérisée par le fait qu'elle comporte, dans un support pharmaceutiquement acceptable, au moins un composé de formule (I) et/ou un de ses sels.

Les composés selon l'invention sont généralement administrés à une dose journalière d'environ 0,01 mg/kg à 100 mg/kg en poids corporel en 1 ou 3 prises.

L'administration peut être effectuée par voie entérale, parentérale, topique ou oculaire. Par voie entérale, les médicaments peuvent se présenter sous forme de comprimés, de gelules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ioniques ou non ioniques ou polymériques ou de patches polymériques ou d'hydrogels permettant une libération contrôlée.

Ces compositions par voie topique peuvent se présenter soit sous forme anhydre, soit sous forme aqueuse selon l'indication clinique.

Par voie oculaire, ce sont principalement des collyres.

Ces compositions contiennent au moins un composé de formule (I) telle que définie ci-dessus ou un des ses sels, à une concentration de préférence comprise entre 0,001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I), dans les compositions cosmétiques est de préférence comprise entre 0,001 et 3 % en poids.

Les compositions médicamenteuses et cosmétiques selon l'invention peuvent contenir des additifs inertes ou même pharmacodynamiquement ou cosmétiquement actifs ou des combinaisons de ceux-ci et notamment : des agents mouillants, des agents dépigmentants tels que l'hydroquinone, l'acide azélaïque, l'acide caféique, l'acide kojique, des agents émollients, des agents hydratants comme le glycérol, le PEG 400, la thiamorpholinone et ses dérivés ou l'urée ; des agents antiséborrhéiques ou antiacnéiques, tels que la S-carboxyméthylcystéine, la S-benzyl-cystéamine, leurs sels et leurs dérivés, la tioxolone ou le peroxyde de benzoyle ; des antibiotiques comme l'érythromycine et ses esters, la néomycine, la clindamycine et ses esters, les tétracyclines ; les antifongiques tels que le kétoconazole ou les polyméthylène-4,5 isothiazolinones-3 ; des agents favorisant la repousse des cheveux, comme le "Minoxidil" (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione) ; des agents anti-inflammatoires stéroïdiens et non stéroïdiens ; des caroténoïdes et, notamment le β-carotène ; des agents anti-psoriatiques tels que l'anthraline et ses dérivés et les acides eicosa-5,8,11,14-tétraynoïque et eicosa-5,8,11-triynoïque, leurs esters et leurs amides ou des agents anti-irritants tels que des dérivés d'α-hydroxyacides et plus particulièrement les dérivés de l'acide mandélique.

Les compositions selon l'invention peuvent également contenir des agents d'amélioration de la saveur, des agents conservateurs tels que les esters de l'acide parahydroxybenzoïque, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### EXEMPLE 1 : Acide 4-(N-méthyl-5,6,7,8-tétrahydro-5,5,8,8 tétraméthyl-2-naphtylcarboxamidino)benzoïque.

### a) 4-(5,6,7,8-tétrahydro-5,5,8,8-tétraméthyl-2-naphtylcarboxamido) benzoate d'allyle.

Dans un ballon, on introduit 2,8 g (16 mmoles) de 4-aminobenzoate d'allyle, 2,5 ml (16 mmoles) de triéthylamine et 50 ml de T.H.F. On ajoute goutte à goutte une solution de 4,3 g (16 mmoles) de chlorure de 5,6,7,8-tétrahydro-5,5,8,8-tétraméthyl-2-naphtoyle dissous dans 50 ml de T.H.F. et agite à température ambiante pendant 2 heures. On verse le milieu réactionnel dans l'eau, extrait avec de l'éther éthylique, décante la phase organique, sèche sur sulfate de magnésium, et évapore. On purifie le résidu obtenu, par chromatographie sur colonne de silice en éluant avec un mélange de dichlorométhane et d'hexane (60-40). Après évaporation des solvants, on obtient 5,7 g de l'ester attendu de point de fusion : 148-149°C.

### b) 4-(α-chloro-5,6,7,8-tétrahydro-5,5,8,8-tétraméthyl-2-naphtyl méthylimino)benzoate d'allyle.

Dans un ballon, on introduit 3,9 g (10mmoles) de l'ester obtenu à l'exemple 1(a), 50 ml de chlorure de thionyle et chauffe à reflux durant 24 heures. On évapore à sec le milieu réactionnel et recueille 4,4 g (100 %) de produit brut attendu, qui est utilisé tel quel pour la suite de la synthèse.

### c) 4-(N-méthyl-5,6,7,8-tétrahydro-5,5,8,8-tétraméthyl-2-naphtylcarboxamidino) benzoate d'allyle.

Dans un ballon, on introduit 2,2 g (0,05 mole) de l'iminochlorure obtenu ci-dessus en 1(b) et tout en refroidissant à 0°C, on ajoute goutte à goutte 50 ml de méthylamine (40 % dans l'eau). On agite à température ambiante une heure, verse le milieu réactionnel dans l'eau, extrait avec de l'éther éthylique, décante la phase organique, sèche sur sulfate de magnésium, et évapore. Le résidu obtenu est purifié par chromatographie sur colonne de silice, élué avec un mélange de dichlorométhane et d'éther éthylique (90-10). Après évaporation des solvants, on obtient 1,6 g (79 %) d'une huile légèrement jaune.

### d) Acide 4-(N-méthyl-5,6,7,8-tétrahydro-5,5,8,8-tétraméthyl-2-naphtylcarboxamidino)benzoïque.

Dans un ballon, on introduit 1,4 g (3,4 mmoles) de l'ester allylique obtenu ci-dessus en (c) et 50 ml de T.H.F. Sous azote on introduit 400 mg (0,35 mmole) de tétrakis (triphénylphosphine) palladium (0) et ajoute goutte à goutte 3 ml (34 mmoles) de morpholine. On agite à température ambiante 4 heures et évapore à sec le milieu réactionnel. On reprend par l'eau le résidu obtenu, acidifie à pH = 5 avec de l'acide chlorhydrique (1N), filtre le solide et le sèche sur pentoxyde de phosphore. Le solide est purifié par chromatographie sur colonne de silice, en éluant avec un mélange de dichlorométhane et de méthanol (80-20). On obtient 850 mg (68 %) d'acide attendu de point de fusion : 164-167°C (avec décomposition).

### EXEMPLE 2 : Acide 4-(5,6,7,8-tétrahydro-5,5,8,8-tétraméthyl-2-naph tylcarboxamidino)benzoïque

### a) 4-(5,6,7,8-tétrahydro-5,5,8,8-tétraméthyl-2-naphtylcarboxamidino) benzoate d'allyle.

De manière analogue à l'exemple 1(c) par réaction de 22 g (0,05 mole) de l'iminochlorure obtenu à l'exemple 1(b) avec 50 ml d'ammoniaque (33 %), on obtient 1,8 g (92 %) de l'ester allylique attendu sous forme d'une huile jaune.

### b) Acide 4-(5,6,7,8-tétrahydro-5,5,8,8-tétraméthyl-2-naphtylcarboxamidino) benzoïque.

De manière analogue à l'exemple 1(d) à partir de 1,6 g (4,1 mmoles) de 4-(5,6,7,8-tétrahydro-5,5,8,8-tétraméthyl-2-naphtylcarboxamidino) benzoate d'allyle, on obtient 710 mg (51 %) d'acide attendu de point de fusion : 192-195°C (avec décomposition).

### EXEMPLE 3 : Acide 4-[5-(1-adamantyl)-2-hydroxy-4-méthoxy benzylidèneamino]benzoïque.

### a) 2-hydroxy-4-méthoxybenzaldéhyde.

Dans un ballon, on introduit 6 g (0,2 mole) d'hydrure de sodium (80 % dans l'huile) et 50 ml de D.M.F. On ajoute goutte à goutte une solution de 27,6 g (0,2 mole) de 2,4-dihydroxybenzaldéhyde dans 100 ml de D.M.F. et agite jusqu'à cessation du dégagement gazeux. On ajoute ensuite, goutte à goutte, 12,5 ml (0,2 mole) d'iodure de méthyle et agite à température ambiante 12 heures. On verse le milieu réactionnel dans l'eau, extrait avec de l'éther éthylique, décante la phase organique, sèche sur sulfate de magnésium, et évapore. Le résidu obtenu est purifié par chromatographie sur colonne de silice en éluant avec un mélange de dichlorométhane et d'hexane (70-30). Après évaporation des solvants, on recueille 20,3 g (68 %) du produit attendu de point de fusion : 38-40°C.

### b) 5-(1-adamantyl)-2-hydroxy-4-méthoxybenzaldéhyde.

Dans un ballon, on introduit 10,4 g (68 mmoles) de l'aldéhyde obtenu ci-dessus en 3(a), 10 g (68 mmoles) de 1-adamantanol et 300 ml de dichlorométhane. On ajoute goutte à goutte 3,6 ml d'acide sulfurique et agite à température ambiante pendant 24 heures. On verse le milieu réactionnel dans l'eau, extrait avec du dichlorométhane, décante la phase organique, lave à l'eau, sèche sur sulfate de magnésium, et évapore. On triture le résidu dans l'éthanol, filtre et sèche le solide obtenu sous vide. On recueille 14,8 g (76 %) du produit attendu de point de fusion : 191-193°C.

### c) Acide 4-[5-(1-adamantyl)-2-hydroxy-4-méthoxybenzylidèneamino] benzoïque

Dans un ballon, on introduit 2,86 g (0,01 mole) de l'aldéhyde obtenu ci-dessus en (b), 1,37 g (0,01 mole) d'acide 4-aminobenzoïque et 500 ml d'éthanol. On ajoute 540 mg (0,01 mole) de méthylate de sodium, chauffe à reflux et distille l'éthanol. On reprend par l'eau, ajuste à pH = 5 avec de l'acide citrique, filtre le solide obtenu, le lave à l'eau et sèche sur pentoxyde de phosphore. On triture le solide dans 50 ml d'éther éthylique et après filtration, recueille 2,9 g (72 %) d'acide attendu de point de fusion : 333-335°C.

### EXEMPLE 4 : Acide 4-[3-(1-adamantyl)-4-méthoxbenzamidino]benzoïque.

### a) 4-[3-(1-adamantyl)-4-méthoxybenzamido] benzoate d'allyle.

De manière analogue à l'exemple 1(a) à partir de 12,6 g (71 mmoles) de 4-aminobenzoate d'allyle et de 21,6 g (71 mmoles) de chlorure de 3-(1-adamantyl)-4-méthoxybenzoyle, on isole 32 g d'amide attendu de point de fusion : 191-192°C.

### b) 4-[α-chloro-3-(1-adamantyl)-4-méthoxyphénylméthylimino]benzoate d'allyle.

A partir de 8,9 g (19,2 mmoles) du dérivé obtenu ci-dessus en (a), on réalise la synthèse suivant le procédé de l'exemple 1(b). Le produit brut obtenu est lavé à l'éther et on obtient 6,5 g (73 %) de 4-[α-chloro-3-(1-adamantyl)-4-méthoxyphénylméthylimino] benzoate d'allyle de point de fusion : 108-110°C.

### c) 4-[3-(1-adamantyl)-4-méthoxybenzamidino]benzoate d'allyle.

De manière analogue à l'exemple 1(c), par réaction de 5 g (107 mmoles) de l'iminochlorure obtenu ci-dessus en (b) avec 10 ml d'ammoniaque à 33 % et après chromatographie du produit brut sur colonne de silice en éluant avec un mélange d'acétate d'éthyle et d'éther de pétrole (40-60), on obtient 2,4 g (51 %) du produit attendu de point de fusion : 173-174°C.

### d) Acide 4-[3-(1-adamantyl)-4-méthoxybenzamidino]benzoïque.

De manière analogue à l'exemple 1(d) à partir de 2 g (4,5 mmoles) de 4- 3-(1-adamantyl)-4-méthoxybenzamidino benzoate d'allyle, on obtient 950 mg (52 %) d'acide attendu de point de fusion : 263-265°C.

### EXEMPLE 5 : Chlorhydrate de l'acide 4-[N-méthyl-3-(1-adamantyl)-4-méthoxybenzamidino]benzoïque.

### a) 4-[N-méthyl-3-(1-adamantyl)-4-méthoxybenzamidino]benzoate d'allyle.

De manière analogue à l'exemple 1(c), par réaction de 3,8 g (7,5 mmoles) de l'iminochlorure préparé à l'exemple 4(b) avec 10 ml de méthylamine (40 % dans l'eau), après chromatographie sur colonne de silice en éluant avec un mélange d'acétate d'éthyle et d'éther de pétrole (40-60), on obtient 2,3 g (67 %) du produit attendu de point de fusion : 77-79°C.

### b) Chlorhydrate de l'acide 4-[N-méthyl-3-(1-adamantyl)-4-méthoxybenzamidino]benzoïque.

Dans un ballon, on introduit 2 g (4,4 mmoles) de l'ester allylique préparé précédemment et 43 ml de T.H.F. On ajoute sous azote 437 mg (0,44 mmoles) de tétrakis(triphénylphosphine)palladium (0), puis on verse goutte à goutte 8,8 mmoles de base préparée à partir de 1,4 g (8,8 mmoles) de malonate de diéthyle et de 262 mg (8,8 mmoles) d'hydrure de sodium (80 % dans l'huile) dans 30 ml de T.H.F. Après agitation à température ambiante pendant 2 heures, le milieu est acidifié par 20 ml d'acide chlorhydrique 1N. Le précipité recueilli est lavé par de l'éther, puis recristallisé dans l'éthanol. On obtient 1,63 g (81 %) de chlorhydrate de l'acide 4-[N-méthyl-3-(1-adamantyl)-4-méthoxybenzamidino]benzoïque de point de fusion : 205-207°C.

### EXEMPLE 6 : Acide 4-[3-(1-adamantyl)-4-méthoxyphényl formamidinométhylthioéther]benzoïque.

### a) 4-[3-(1-adamantyl)-4-méthoxyphénylformamidinométhyl thioéther] benzoate d'allyle.

Dans un ballon, on met en suspension 700 mg (10 mmoles) de thiométhylate de sodium dans 25 ml de diméthoxyéthane. A température ambiante et sous courant d'azote, on introduit goutte à goutte 3,5 g (7,6 mmoles) de 4-[α-chloro-3-(1-adamantyl)-4-méthoxyphénylméthylimino]benzoate d'allyle obtenu à l'exemple 4(b) et dissous dans 50 ml de D.M.E. Après 3 heures de réaction, le mélange est versé dans l'eau, et extrait avec de l'éther éthylique. La phase organique est séchée sur sulfate de sodium, puis évaporée sous vide : le produit brut obtenu est chromatographié sur colonne de silice élué avec un mélange d'éther éthylique et d'hexane (20-80). On obtient après évaporation des solvants 2,6 g (73 %) de produit attendu de point de fusion : 50-53°C.

### b) Acide 4-[3-(1-adamantyl)-4-méthoxyphénylformamidinométhylthio éther]benzoïque.

De manière analogue à l'exemple 5(b) à partir de 1,5 g (3,2 mmoles) d'ester allylique obtenu ci-dessus en (a) on obtient 1,05 g (77 %) d'acide 4-[3-(1-adamantyl)-4-méthoxyphénylformamidinométhylthioéther] benzoïque de point de fusion : 235-237°C.

### EXEMPLE 7 : Acide 4-[3-(1-adamantyl)-4-méthoxyphényl formamidinométhyléther]benzoïque.

### a) 4-[3-(1-adamantyl)-4-méthoxyphénylformamidinométhyléther] benzoate d'allyle.

Dans un tricol, on introduit, sous atmosphère inerte, 30 ml de dichlorométhane, 1,43 ml (10 mmoles) de triéthylamine et 324 µl (8 mmoles) de méthanol. Le mélange est porté à 50°C, puis on ajoute goutte à goutte 3,7 g (8 mmoles) d'iminochlorure obtenu à l'exemple 4(b) dissous dans 35 ml de dichlorométhane. A la fin de l'addition, on rajoute un équivalent de méthanol, soit 324 µl. Après 4 heures de réaction, le mélange réactionnel est versé dans l'eau, puis extrait par le dichlorométhane. La phase organique est décantée, séchée sur sulfate de magnésium et évaporée à sec. Le résidu liquide obtenu est chromatographié sur colonne de silice en éluant à l'aide d'un mélange d'éther éthylique et d'hexane (10-90). On récupère après évaporation des solvants, 1,7 g (46 %) de produit attendu de point de fusion : 85-87°C.

### b) Acide 4-[3-(1-adamantyl)-4-méthoxyphénylformamidinométhyléther] benzoïque.

De manière analogue à l'exemple 5(b) à partir de 1,08 g (2,6 mmoles) d'ester allylique obtenu ci-dessus en (a), on obtient 720 mg (67 %) d'acide 4-[3-(1-adamantyl)-4-méthoxyphénylformamidinométhyléther]benzoïque de point de fusion : 224-226°C.

### EXEMPLE 8 : 4-[3-(1-adamantyl)-4-méthoxybenzylidèneamino]benzoate de méthyle.

3 g d'alumine basique et 1,94 g (12,9 mmoles) de 4-aminobenzoate de méthyle sont mélangés au mortier, puis introduits dans un ballon. On ajoute sous agitation 3 g (11,1 mmoles) de 3-(1-adamantyl)-4-méthoxy benzaldéhyde dans 50 ml de dichlorométhane anhydre. Après chauffage à reflux pendant 24 heures, le milieu réactionnel est extrait par le dichlorométhane, et évaporé à sec. Le produit brut est recristallisé dans un mélange d'acétate d'éthyle et d'acétone. Après filtration, on obtient 3,4 g (75 %) de produit attendu de point de fusion : 180-182°C.

### EXEMPLE 9 : Acide 4-[3-(1-adamantyl)-4-méthoxbenzylidèneamino] benzoïque.

### a) 4-[3-(1-adamantyl)-4-méthoxybenzylidèneamino]benzoate d'allyle.

A partir de 3,4 g (12,6 mmoles) de 3-(1-adamantyl)-4-méthoxy benzaldéhyde et de 2,6 g (14,4 mmoles) de 4-aminobenzoate d'allyle on réalise la synthèse suivant le procédé décrit à l'exemple 8. Le produit brut obtenu est recristallisé dans l'acétate d'éthyle. Après filtration et séchage, on obtient 4,1 g (76 %) d'ester allylique de point de fusion : 160-162°C.

### b) Acide 4-[3-(1-adamantyl)-4-méthoxybenzylidèneamino]benzoïque.

De manière analogue à l'exemple 1(d) à partir de 2 g (4,7 mmoles) d'ester allylique préparé ci-dessus en (a), on obtient 1,4 g (77 %) d'acide 4-[3-(1-adamantyl)-4-méthoxybenzylidèneamino]benzoïque de point de fusion : 299-301°C.

### EXEMPLE 10

### Acide 4-(3-tert-butyl-4-méthoxybenzamidino)benzoïque

### a) 4-(3-tert-butyl-4-méthoxybenzamido)benzoate d'allyle

Dans un ballon, on introduit 5,92 g (33,4 mmoles) de 4- aminobenzoate d'allyle, 5,6 ml (40,25 mmoles) de triéthylamine et 100 ml de T.H.F. On ajoute goutte à goutte une solution de 8 g (38,4 mmoles) de chlorure de 3-tert-butyl-4- méthoxybenzoyle dissous dans 50 ml de T.H.F. et agite à température ambiante pendant 4 heures. On verse le milieu réactionnel dans l'eau, extrait avec du dichlorométhane, décante la phase organique, lave à l'eau déminéralisée jusqu'à pH 6, sèche sur Na2SO4, filtre et évapore les solvants sous pression réduite. On cristallise l'huile marron obtenue dans l'hexane, filtre le solide et le recristallise dans l'éthanol absolu. Après filtration sur verre fritté et séchage 48 heures à 80°C, on obtient 5,1 g (42,5%) d'ester attendu, sous forme de cristaux blancs, de point de fusion: 180-182 °C.

### b) 4-(α-chloro-3-tert-butyl-4-méthoxyphénylméthylimino)benzoate d'allyle

A partir de 2 g (5,44 mmoles) du dérivé obtenu ci-dessus (a), on réalise la synthèse suivant le procédé de l'exemple 1(b). Après évaporation à sec, on recueille 2,3 g (100%) de produit brut attendu qui est utilisé tel quel pour la suite de la synthèse.

### c) 4-(3-tert-butyl-4-méthoxybenzamidino)benzoate d'allyle

De manière analogue à l'exemple 1(c), par réaction de 2,3 g (5,44 mmoles) de l'iminochlorure obtenu ci-dessus (b) avec 40 ml d'ammoniaque à 33%, puis extraction par l'acétate d'éthyle, chromatographie sur colonne de silice en éluant avec un mélange d'acétate d'éthyle et d'hexane (50/50), on obtient 1,06 g (53%) de produit attendu, de point de fusion: 120-122°C.

### d) Acide 4-(3-tert-butyl-4-méthoxybenzamidino)benzoïque

Dans un ballon, on introduit 300 mg (0,82 mmole) de dérivé obtenu ci-dessus (c), 180 mg (4,5 mmoles) de soude et 10 ml de méthanol. Après 24 heures de réaction au reflux du méthanol, on évapore et reprend par l'eau. On neutralise à pH 5-6 par l'acide acétique, filtre le précipité obtenu, lave à l'eau déminéralisée, puis sèche 48 heures à 80°C. On obtient 210 mg (78%) d'acide attendu, de point fusion: 170-172°C.

### EXEMPLE 11

### 4-(5,6,7,8-tétrahydro-5,5,8,8-tétraméthyl-2-naphtylcarboxamidino)benzoate de méthyle

### a) 4-(α-chloro-5,6,7,8-tétrahydro-5,5,8,8-tétraméthyl-2- naphtylméthylimino)benzoate de méthyle

A partir de 4,94 g (13,5 mmoles) de 4-(5,6,7,8-tétrahydro-5,5,8,8-tétraméthyl-2-naphtylcarboxamido)benzoate de méthyle, on réalise la synthèse suivant le procédé de l'exemple 1(b). Après évaporation à sec, on recueille 5,7 g (100%) de produit brut attendu qui est utilisé tel quel pour la suite de la synthèse.

### b) 4-(5,6,7,8-tétrahydro-5,5,8,8-tétraméthyl-2-naphtylcarboxamidino)benzoate de méthyle

De manière analogue à l'exemple 1(c) par réaction de 5,7 g (13,5 mmoles) d'iminochlorure obtenu ci-dessus (a) avec 125 ml d'ammoniaque à 33%, on recueille un produit brut huileux qui est chromatographié sur colonne de silice dans le système acétate d'éthyle-hexane (30/70). On récupère ainsi 600 mg (12%) d'ester attendu, de point de fusion: 196-198°C.

### EXEMPLE 12

### Alcool 4-(5,6,7,8-tétrahydro-5,5,8,8-tétraméthyl-2-naphtylcarboxamidino)benzylique

Dans un ballon, on introduit 420 mg (1,15 mmole) d'ester obtenu à l'exemple 11(b) et 25 ml de T.H.F. On introduit par portions 110 mg (2,88 mmoles) d'hydrure double de lithium et d'aluminium, laisse agiter 8 heures à température ambiante puis hydrolyse le milieu réactionnel par du surate de sodium hydraté. On filtre le précipité obtenu et évapore le filtrat. On obtient ainsi 360 mg (93%) d'alcool attendu, de point de fusion: 171-172 °C.

### EXEMPLE 13

### 4-(5,6,7,8-tétrahydro-5,5,8,8-tétraméthyl-2-naphtylcarboxamidino) toluène

### a) 4-(α-chloro-5,6,7,8-tétrahydro-5,5,8,8-tétraméthyl-2- naphtylméthylimino)toluène

A partir de 2 g (6,22 mmoles) de 4-(5,6,7,8-tétrahydro-5,5,8,8-tétraméthyl-2-naphtylcarboxamido) toluène, on réalise la synthèse suivant le procédé de l'exemple 1(b). Après évaporation à sec, on recueille 2,34 g (100%) de produit brut attendu qui est utilisé tel quel dans la suite de la synthèse.

### b) 4-(5,6,7,8-tétrahydro-5,5,8,8-tétraméthyl-2-naphtylcarboxamidino)toluène

De manière analogue à l'exemple 1(c) par réaction de 2,34 g (6,22 mmoles) d'iminochlorure obtenu ci-dessus (a) avec 60 ml d'ammoniaque à 33%, puis extraction par l'acétate d'éthyle et chromatographie sur colonne de silice dans le système d'éluant acétate d'éthyle-hexane (40/60), on obtient 740 mg (37%) de produit attendu, de point de fusion: 147-149°C.

### EXEMPLE 14

### 4-(5,6,7,8-tétrahydro-5,5,8,8-tétraméthyl-2-naphtylcarboxamidino)benzamide

### a) Chlorure de 4-(α-chloro-5,6,7,8-tétrahydro-5,5,8,8-tétraméthyl-2-naphtylméthylimino)benzoyle

Dans un ballon, on introduit 1 g (2,84 mmoles) d'acide 4-(5,6,7,8-tétrahydro-5,5,8,8-tétraméthyl-2-naphtylcarboxamidino)benzoïque et 10 ml de chlorure de thionyle. On chauffe à reflux durant 24 heures, évaporé à sec et récupère 1,2 g (100%) de produit brut attendu utilisé tel quel pour la suite de la synthèse.

### b) 4-(5,6,7,8-tétrahydro-5,5,8,8-tétraméthyl-2-naphtylcarboxamidino)benzamide

Dans un ballon, on introduit 1,2 g (2,84 mmoles) du dérivé obtenu ci-dessus (a) et 15 ml de T.H.F. On agite en maintenant le milieu réactionnel à 0°C et verse goutte à goutte 40 ml d'ammoniaque à 33%. On laisse 4 heures sous agitation, puis extrait par l'acétate d'éthyle, lave la phase organique jusqu'à neutralité, sèche sur sulfate de sodium, filtre et évapore les solvants sous pression réduite. On recueille 1 g de produit brut qui est repris dans l'acétate d'éthyle chaud. Après filtration, on obtient ainsi 470 mg (47%) d'amide attendu de point de fusion : 247-249°C.

### EXEMPLE 15

### Acide 2-hydroxy-4-(5,6,7,8-tétrahydro-5,5,8,8-tétraméthyl-2-naphtylcarboxamidino) benzoïque

### a) 2-hydroxy-4-aminobenzoate d'allyle

Dans un ballon, on introduit 50 ml d'alcool allylique et 460 mg (0,02 mole) de sodium en refroidissant avec un bain de glace. On ajoute ensuite une solution de 8,3 g (0,05 mole) de 2-hydroxy-4-aminobenzoate de méthyle dans 100 ml d'alcool allylique et chauffe tout en distillant le méthanol formé. On évapore à sec, reprend par de l'eau, acidifie avec de l'acide chlorhydrique (1N), extrait avec de l'éther éthylique, décante la phase organique, sèche sur sulfate de magnésium et évapore. On reprend dans l'hexane, filtre et sèche. On recueille 8,6 g (90%) d'ester allylique de point de fusion : 58-59°C.

### b) 2-hydroxy-4-(5,6,7,8-tétrahydro-5,5,8,8-tétraméthyl-2-naphtylcarboxamido)benzoate d'allyle

De manière analogue à l'exemple 1(a) par réaction de 6,7 g (34,7 mmoles) de 2-hydroxy-4-aminobenzoate d'allyle avec 8,7 g (34,7 mmoles) de chlorure de 5,6,7,8-tétrahydro-5,5,8,8- tétraméthyl-2-naphtoyle, on obtient 12,7 g (90%) de l'ester allylique attendu, qui fond à 129-130°C.

### c) 2-hydroxy-4-[α-chloro-(5,6,7,8-tétrahydro-5,5,8,8-tétraméthyl-2-naphtyl) méthylimino]benzoate d'allyle

Dans un ballon, on introduit 4 g (0,01 mole) du produit précédent et 50 ml de chlorure de thionyle et chauffe à reflux durant 24 heures. On évapore à sec le milieu réactionnel et recueille 4,2 g (100%) de produit brut attendu, qui est utilisé tel quel pour la suite de la synthèse.

### d) 2-hydroxy-4-(5,6,7,8-tétrahydro-5,5,8,8-tétraméthyl-2-naphtylcarboxamidino)benzoate d'allyle

De manière analogue à l'exemple 1(c), par réaction de 4,2 g (10 mmoles) de l'iminochlorure précédent avec 50 ml d'ammoniaque (33%), on obtient 2,1 g (52%) de l'ester allylique attendu, sous forme d'une huile jaune.

### e) Acide 2-hydroxy-4-(5,6,7,8-tétrahydro-5,5,8,8-tétraméthyl-2-naphtylcarboxamidino) benzoïque

De manière analogue à l'exemple 1(d) à partir de 2,1 g (5,2 mmoles) de 2-hydroxy-4-(5,6,7,8-tétrahydro-5,5,8,8- tétraméthyl-2-naphtylcarboxamidino)benzoate d'allyle, on obtient 1,1 g (58%) d'acide attendu, de point de fusion: 209-210°C.

### EXEMPLE 16

### 4-(α-chloro-5,6,7,8-tétrahydro-5,5,8,8-tétraméthyl-2-naphtylméthylimino)benzoate d'allyle

Dans un ballon, on introduit 40,5 g (0,103 mole) de 4-(5,6,7,8-tétrahydro-5,5,8,8-tétraméthyl -2-naphtylcarboxamido)benzoate d'allyle, 500 ml de chlorure de thionyle et chauffe à reflux pendant 48 heures. On évapore à sec le milieu réactionnel. Le résidu est cristallisé dans l'hexane, filtré, lavé à l'hexane et séché sous vide. On recueille 16,86 g (47%) du produit attendu, de point de fusion : 80°C.

### EXEMPLE 17

### Acide 4-(N¹-phényl-5,6,7,8-tétrahydro-5,5,8,8-tétraméthyl-2- naphtylcarboxamidino) benzoïque

### a) 4-(N¹-phényl-5,6,7,8-tétrahydro-5,5,8,8-tétraméthyl-2- naphtylcarboxamidino)benzoate d'allyle

Dans un ballon on introduit 4,08 ml (44,8 mmoles) d'aniline et 10 ml de THF. On ajoute, goutte à goutte, 4 g (8,97 mmoles) de l'iminochlorure obtenu à l'exemple 16, dissous dans 40 ml de THF. On agite à température ambiante pendant 24 heures, on verse le milieu réactionnel dans l'eau, acidifie à pH 5 avec de l'acide chlorhydrique (1N), extrait avec de l'acétate d'éthyle, lave à l'eau, décante la phase organique, sèche sur sulfate de sodium et évapore. Le résidu obtenu est purifié par chromatographie sur colonne de silice, élué avec un mélange d'hexane et d'acétate d'éthyle (85/15). Après évaporation des solvants, on obtient 3,6 g (87%) d'une huile jaune.

### b) Acide 4-(N¹-phényl-5,6,7,8-tétrahydro-5,5,8,8-tétraméthyl-2-naphtylcarboxamidino) benzoïque

Dans un ballon, on introduit 3,55 g (7,62 mmoles) de l'ester allylique obtenu à l'exemple 17(a) et 70 ml de THF. On ajoute 880 mg (0,76 mmole) de tétrakis (triphénylphosphine) palladium (0) et ajoute goutte-à-goutte 6,64 ml (76,2 mmoles) de morpholine. On agite à température ambiante pendant 1 heure, verse le milieu réactionnel dans l'eau, acidifie à pH 5 avec de l'acide acétique, extrait avec de l'acétate d'éthyle, lave à l'eau, sèche sur sulfate de sodium et évapore. Le résidu obtenu est purifié par chromatographie sur colonne de silice, élué avec un mélange d'acétate d'éthyle et d'hexane (50/50). Après évaporation des solvants, on obtient 3,05 g (95%) d'acide attendu, de point de fusion: 125-130°C.

### EXEMPLE 18

### Chlorhydrate de l'acide 4-(N¹-phényl-5,6,7,8-tétrahydro-5,5,8,8-tétraméthyl-2-naphtylcarboxamidino) benzoïque

Dans un ballon, on introduit 500 mg (1,17 mmole) de l'acide obtenu à l'exemple 17(b) et 15 ml d'acétone. On ajoute 1,12 ml d'acide chlorhydrique (1,045N). On filtre le précipité, lave à l'acétone et sèche. On recueille 490 mg (90%) du chlorhydrate attendu, de point de fusion: 297°C (avec décomposition).

### EXEMPLE 19

### Acide 4-(N¹-benzyl-5,6,7,8-tétrahydro-5,5,8,8-tétraméthyl-2-naphtylcarboxamidino) benzoïque

### a) 4-(N¹-benzyl-5,6,7,8-tétrahydro-5,5,8,8-tétraméthyl-2- naphtylcarboxamidino)benzoate d'allyle

De manière analogue à l'exemple 17(a), à partir de 5 g (11,2 mmoles) d'iminochlorure obtenu à l'exemple 16 et 5,67 ml (52 mmoles) de benzylamine, on obtient 3,5 g (67,3%) de l'ester allylique attendu sous forme d'une huile jaune.

### b) Acide 4-(N¹-benzyl-5,6,7,8-tétrahydro-5,5,8,8-tétraméthyl-2-naphtyl -carboxamidino)benzoïque

De manière analogue à l'exemple 17(b), à partir de 3,5 g (7,3 mmoles) de l'ester allylique obtenu à l'exemple 19(a), on obtient après chromatographie sur colonne de silice, élué avec un mélange d'acétate d'éthyle et d'hexane (60/40) et évaporation des solvants, 2,45 g (76,6%) d'acide attendu, de point de fusion : 105°C.

### EXEMPLE 20

### Acide 4-(N¹,N¹-diméthyl-5,6,7,8-tétrahydro-5,5,8,8-tétraméthyl 2-naphtyl carboxamidino)benzoïque

### a) 4-(N¹,N¹-diméthyl-5,6,7,8-tétrahydro-5,5,8,8-tétraméthyl-2-naphtyl carboxamidino)benzoate d'allyle

De manière analogue à l'exemple 17(a), à partir de 4 g (8,97 mmoles) d'iminochlorure obtenu à l'exemple 16 et 5,63 ml (0,452 mole) de diméthylamine (40% dans l'eau), on obtient 3,33 g (89%) de l'ester allylique attendu, sous forme d'une huile jaune.

### b) Acide 4-(N¹,N¹-diméthyl-5,6,7,8-tétrahydro-5,5,8,8-tétraméthyl-2-naphtyl carboxamidino)benzoïque

De manière analogue à l'exemple 17(b), à partir de 3,3 g (7,92 mmoles) de l'ester allylique obtenu à l'exemple 20(a), on obtient 2,5 g (83%) d'acide attendu de point de fusion 240°C.

### EXEMPLE 21

### Acide 4-[N²-(5,6,7,8-tétrahydro-5,5,8,8-tétraméthyl-2- naphtyl)amidino]benzoïque

### a) 4-[N²-(5,6,7,8-tétrahydro-5,5,8,8-tétraméthyl-2- naphtyl)amidino]benzoate de méthyle

De manière analogue à l'exemple 11(b), par réaction de 2,5 g (6,9 mmoles) de 4-(5,6,7,8-tétrahydro-5,5,8,8-tétraméthyl-2- naphtylcarbamoyl)benzoate de méthyle avec 25 ml de chlorure de thionyle, on recueille, apis évaporation, l'iminochlorure correspondant qui est directement mis à réagir avec 100 ml d'ammoniaque à 33%. Après recristallisation dans un mélange d'acétate d'éthyle et d'hexane, on obtient 1,5 g (59,8%) d'ester attendu, de point de fusion : 218-220°C.

### b) Acide 4-[N²-(5,6,7,8-tétrahydro-5,5,8,8-tétraméthyl-2- naphtyl)amidino]benzoïque

De manière analogue à l'exemple 10(d), à partir de 500 mg (1,37 mmole) d'ester obtenu précédemment en (a), on obtient 300 mg (62%) d'acide attendu, de point de fusion: 281-284°C.

### EXEMPLE 22

### Acide 4-[N¹-phényl-N²-(5,6,7,8-tétrahydro-5,5,8,8-tétraméthyl 2-naphtyl)amidino]benzoique

### a) 4-[N¹-phényl-N²-(5,6,7,8-tétrahydro-5,5,8,8-tétraméthyl-2- naphtyl)amidino]benzoate de méthyle

De manière analogue à l'exemple 21(a), on réalise la synthèse à partir de 2,9 g (6,9 mmoles) d'iminochlorure préparé en 21(a) et 3,2 ml (34,5 mmoles) d'aniline. Après chromatographie sur colonne de silice dans un mélange d'acétate d'éthyle et d'hexane (20/80) et lavage dans l'hexane chaud, on isole 390 mg (13%) d'ester attendu, de point de fusion : 163-164°C.

### b) Acide 4-[N¹-phényl-N²-(5,6,7,8-tétrahydro-5,5,8,8-tétraméthyl-2-naphtyl)amidino] benzoïque

De manière analogue à l'exemple 10(d) et à partir de 300 mg (0,68 mmole) de dérivé obtenu ci-dessus (a), on isole 230 mg (79%) d'acide attendu, de point de fusion: 281-284°C.

### EXEMPLE 23

### Chlorhydrate du 4-(5,6,7,8-tétrahydro-5,5,8,8-tétraméthyl-2- naphtylcarboxamidino)phénol

### a) 4-(α-chloro-5,6,7,8-tétrahydro-5,5,8,8-tétraméthyl-2- naphtylméthylimino)anisole

De manière analogue à l'exemple 1(b) par réaction de 3 g (8,9 mmoles) de 4-(5,6,7,8-tétrahydro-5,5,8,8-tétraméthyl-2- naphtylcarboxamido)anisole avec 30 ml de chlorure de thionyle, on recueille l'iminochlorure attendu utilisé tel quel pour la suite de la synthèse.

### b) 4-(5,6,7,8-tétrahydro-5,5,8,8-tétraméthyl-2-naphtylcarboxamidino)anisole

De manière analogue à l'exemple 1(c), par réaction de 8,9 mmoles d'iminochlorure obtenu ci-dessus (a) avec 62 ml d'ammoniaque à 33% et après recristallisation dans l'hexane, on isole 1,22 g (40%) de produit attendu, de point de fusion: 138-140°C.

### c) Chlorhydrate du 4-(5,6,7,8-tétrahydro-5,5,8,8-tétraméthyl-2-naphtylcarboxamidino)phénol

Dans un ballon, on introduit 800 mg (2,38 mmoles) du dérivé obtenu précédemment en (b) et 5 g (43,3 mmoles) de chlorhydrate de pyridine. On porte le mélange réactionnel progressivement à 190-200°C sous vive agitation et maintient cette température pendant 1 heure. On laisse revenir à 100°C et ajoute un large excès d'eau. On filtre le précipité obtenu et lave abondamment à l'eau déminéralisée, sèche 48 heures à 80°C et obtient 500 mg (59%) de phénol attendu, de point de fusion : 255-256°C.

### EXEMPLE 24

### Acide 4-(N¹-méthyl-N²-méthyl-5,6,7,8-tétrahydro-5,5,8,8-tétraméthyl -2-naphtylcarboxamidino)benzoïque

### a) N-méthyl(5,6,7,8-tétrahydro-5,5,8,8-tétraméthyl-2-naphtyl)amide

Dans un ballon, on introduit 10,75 g (43 mmoles) de chlorure de 5,6,7,8-tétrahydro-5,5,8,8-tétraméthyl-2-naphtoyle et 70 ml de T.H.F. On ajoute goutte à goutte 10 ml de N-méthylamine (à 40 % dans l'eau) et laisse agiter 8 heures à température ambiante. On jette dans l'eau, filtre le précipité, lave jusqu'à neutralité et sèche 24 heures à 80°C, on obtient ainsi 10,16 g (96,5 %) d'amide attendu de point de fusion 136-140°C.

### b) 4-(N¹-méthyl-N²-méthyl-5,6,7,8-tétrahydro-5,5,8,8-tétraméthyl-2-napthylcarboxamidino) benzoate de méthyle

De manière analogue à l'exemple 11(b) par réaction de 1,5 g (6,12 mmoles) du dérivé obtenu précédemment (a), avec 20 ml de chlorure de thionyle, on recueille, après évaporation l'iminochlorure correspondant que l'on dilue dans 25 ml de T.H.F. On ajoute à température ambiante 2 g ( 12,12 mmoles) de 4-(N-méthylamino)benzoate de méthyle dissous dans 20 ml de T.H.F. et laisse agiter 8 heures. Après chromatographie sur colonne de silice éluée dans l'acétate d'éthyle, on isole 380 mg (16 %) d'ester attendu de point de fusion 225-230°C.

### c) Acide 4-(N¹-méthyl-N²-méthyl-5,6,7,8-tétrahydro-5,5,8,8-tétraméthyl-2-naphtylcarboxamidino) benzoïque

Dans un ballon, on introduit 250 mg (0,64 mmoles) d'ester obtenu précédemment (b), 630 mg (15,8 mmoles) de soude et 12 ml de méthanol. On maintient 4 heures au reflux puis évapore le méthanol, reprend dans l'eau, neutralise par l'acide acétique et évapore à sec. On reprend le résidu par du T.H.F. filtre et évapore le filtrat. On obtient une huile qui précipite dans l'hexane. Après filtration, on isole 35 mg (14,5 %) d'acide attendu de point de fusion 275-280°C.

### EXEMPLES DE FORMULATIONS

### A. VOIE ORALE

### (a) Comprimé de 0,2 g

| | |
|---|---|
| Composé de l'exemple 1 | 0,001 g |
| Amidon | 0,114 g |
| Phosphate bicalcique | 0,020 g |
| Silice | 0,020 g |
| Lactose | 0,030 g |
| Talc | 0,010 g |
| Stéarate de magnésium | 0,005 g |

Dans cet exemple, le composé de l'exemple 1 peut être remplacé par la même quantité du composé de l'exemple 2.

### (b) Suspension buvable en ampoules de 5 ml

| | |
|---|---|
| Composé de l'exemple 3 | 0,500 g |
| Glycérine | 0,500 g |
| Sorbitol à 70 % | 0,500 g |
| Saccharinate de sodium | 0,010 g |
| Parahydroxybenzoate de méthyle Arôme qs | 0,040 g |
| Eau purifiée qsp | 5 ml |

Dans cet exemple, le composé de l'exemple 3 peut être remplacé par la même quantité du composé de l'exemple 4.

### (c) Comprimé de 0,8 g

| | |
|---|---|
| Composé de l'exemple 7 | 0,500 g |
| Amidon prégélatinisé | 0,100 g |
| Cellulose microcristalline | 0,115 g |
| Lactose | 0,075 g |
| Stéarate de magnésium | 0,010 g |

Dans cet exemple, le composé de l'exemple 7 peut être remplacé par la même quantité du composé de l'exemple 9.

### (d) Suspension buvable en ampoules de 10 ml

| | |
|---|---|
| Composé de l'exemple 5 | 0,200 g |
| Glycérine | 1,000 g |
| Sorbitol à 70 % | 1,000 g |
| Saccharinate de sodium | 0,010 g |
| Parahydroxybenzoate de méthyle Arôme qs | 0,080 g |
| Eau purifiée qsp | 10 ml |

### B. VOIE TOPIQUE

### (a) Onguent

| | |
|---|---|
| Composé de l'exemple 1 | 0,020 g |
| Myristate d'isopropyle | 81,700 g |
| Huile de vaseline fluide | 9,100 g |
| Silice vendue par la Société DEGUSSA sous la dénomination "Aérosil 200" | 9,180 g |

Dans cet exemple, le composé de l'exemple 1 peut être remplacé par la même quantité du composé de l'exemple 2.

### (b) Onguent

| | |
|---|---|
| Composé de l'exemple 3 | 0,300 g |
| Vaseline blanche codex qsp | 100 g |

Dans cet exemple, le composé de l'exemple 3 peut être remplacé par la même quantité du composé de l'exemple 4.

### (c) Crème Eau-dans-l'Huile non ionique

| | |
|---|---|
| Composé de l'exemple 7 | 0,100 g |
| Mélange d'alcools de lanoline émulsifs, de cires et d'huiles raffinés, vendu par la Société BDF sous la dénomination "Eucerine anhydre" | 39,900 g |
| Parahydroxybenzoate de méthyle | 0,075 g |
| Parahydroxybenzoate de propyle | 0,075 g |
| Eau déminéralisée stérile qsp | 100 g |

Dans cet exemple, le composé de l'exemple 7 peut être remplacé par la même quantité du composé de l'exemple 9.

### (d) Lotion

| | |
|---|---|
| Composé de l'exemple 5 | 0,100 g |
| Polyéthylène glycol (PEG 400) | 69,900 g |
| Ethanol 95 % | 30,000 g |

### (e) Onguent hydrophobe

| | |
|---|---|
| Composé de l'exemple 6 | 0,300 g |
| Myristate d'isopropyle | 36,400 g |
| Huile de silicone vendue par la Société Rhône Poulenc sous la dénomination "Rhodorsil 47 V 300" | 36,400 g |
| Cire d'abeille | 13,600 g |
| Huile de silicone vendue par la Société Goldschmidt sous la dénomination "Abil 300.000 cst" qsp | 100 g |

### (f) Crème Huile-dans-l'Eau non ionique

| | |
|---|---|
| Composé de l'exemple 8 | 1,000 g |
| Alcool cétylique | 4,000 g |
| Monostéarate de glycérol | 2,500 g |
| Stéarate de PEG 50 | 2,500 g |
| Beurre de karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile qsp | 100 g |

## Revendications (Revendications pour l'(les) Etat(s) contractant(s) suivant(s): AT, BE, CH, DE, DK, FR, GB, GR, IT, LI, NL, PT, SE)

1. Composés bi-aromatiques, caractérisés par le fait qu'ils répondent à la formule générale suivante : 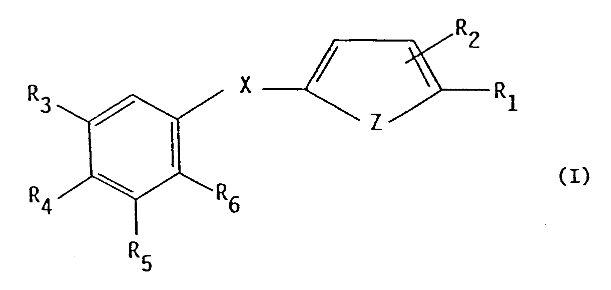 dans laquelle :
R₁ représente un atome d'hydrogène, le groupe OH, le radical -CH₃, le radical -CH₂OH, le radical -COR₇, le radical -CH(OH)CH₃, le radical -CH₂OCOR₈, le radical -SO₂R₉, le radical SOR₉ ou le radical -SR₉,
R₇ représentant un atome d'hydrogène, le groupe OH, le radical -OR₁₀, le radical : 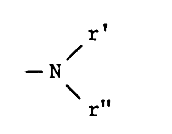 un radical alkyle inférieur, un radical monohydroxyalkyle, un radical polyhydroxyalkyle ou le reste d'un sucre,
R₈ représentant un radical alkyle linéaire ou ramifié, ayant de 1 à 20 atomes de carbone, un radical alkényle ayant de 2 à 20 atomes de carbone ou le reste d'un sucre,
R₉ représentant le groupe OH, un radical alkyle inférieur ou le radical 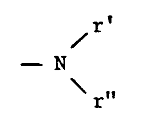 R₁₀ représentant un radical alkyle ayant de 1 à 20 atomes de carbone ou un radical alkényle ayant de 2 à 20 atomes de carbone,
r′ et r˝, identiques ou différents, représentent un atome d'hydrogène, un radical alkyle inférieur, un radical aryle, un radical aralkyle, un reste d'aminoacide, un reste de sucre, un reste de sucre aminé ou un hétérocyle ou r' et r'' pris ensemble forment un hétérocycle,
R₂ et R₆ représentent un atome d'hydrogène, le groupe OH, un radical alkyle inférieur, un radical alkoxy ayant de 1 à 6 atomes de carbone, un atome de fluor, un atome de chlore ou le groupe CF₃,
R₃ et R₅ représentent un radical alkyle α,α'-disubstitué ayant de 4 à 12 atomes de carbone ou un radical cycloalkyle mono ou polycyclique ayant de 5 à 12 atomes de carbone dont le carbone de liaison est trisubstitué,
R₄ représente un atome d'hydrogène, le groupe OH, un radical alkoxy ayant de 1 à 6 atomes de carbone, un radical alkyle α,α'-disubstitué ayant de 4 à 12 atomes de carbone,
R₃ et R₄ ou R₄ et R₅ pris ensemble peuvent former, avec le noyau benzénique adjacent, un cycle à 5 ou 6 atomes de carbone substitués par 2 à 6 groupes méthyles,
Z représente le radical divalent -CH=CR₁₁-
R₁₁ représentant un atome d'hydrogène, le groupe OH ou un radical alkyle inférieur,
X est choisi parmi les radicaux :
(i) - CR₁₃= N -
(ii) - N = C₁₃-
(iii) 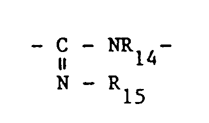 (iv) 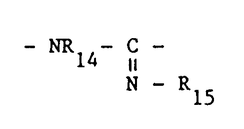
R₁₃ représentant le radical R₁₆, le radical OR₁₆, le radical -SR₁₆ ou le radical : 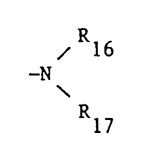 R₁₆ et R₁₇ représentant un atome d'hydrogène, un radical alkyle inférieur, un radical fluoroalkyle inférieur, un radical alkényle ayant de 2 à 6 atomes de carbone, un radical alkynyle ayant de 2 à 6 atomes de carbone, un radical aryle ou un radical aralkyle,
R₁₄ représentant un radical alkyle inférieur,
R₁₅ représentant un radical alkyle inférieur ou un radical fluoroalkyle inférieur,
à l'exclusion des composés de Formule (I) dans laquelle R₃ et R₅ sont identiques lorsque X représente le radical - CR₁₃= N - , R₁₃ représentant un atome d'hydrogène,
et les sels desdits composés de formule (I) obtenus par addition de base lorsque R₁ représente une fonction acide carboxylique ou par addition d'acide.

2. Composés selon la revendication 1, caractérisés par le fait qu'ils se présentent sous forme de sels d'un métal alcalin ou alcalino-terreux ou encore de zinc ou d'une amine organique.

3. Composés selon la revendication 1, caractérisés par le fait qu'ils se présentent sous forme de sels d'un acide minéral ou organique choisi parmi l'acide chlorhydrique, sulfurique, acétique, citrique, fumarique, hémisuccinique, maléique et mandélique.

4. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

5. Composés selon la revendication 1, caractérisés par le fait que le radical alcoxy ayant de 1 à 6 atomes de carbones est pris dans le groupe constitué par un radical méthoxy, éthoxy, isopropoxy et butoxy.

6. Composés selon la revendication 1, caractérisés par le fait que le radical alkyle α,α′-disubstitué est pris dans le groupe constitué par : un radical tert-butyle, 1,1-diméthyl propyle, 1-méthyl 1-éthyl propyle, 1-méthyl 1-éthyl hexyle et 1,1-diméthyl décyle.

7. Composés selon la revendication 1, caractérisés par le fait que le radical cycloalkyle mono ou polycyclique ayant de 5 à 12 atomes de carbone dont le carbone de liaison est trisubstitué, est le radical 1-méthyl cyclohexyle ou 1-adamantyle.

8. Composés selon la revendication 1, caractérisés par le fait que le radical monohydroxyalkyle est un radical 2-hydroxyéthyle, 2-hydroxypropyle ou 3-hydroxypropyle.

9. Composés selon la revendication 1, caractérisés par le fait que le radical polyhydroxyalkyle comporte de 2 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxypentyle et le reste du pentaérythritol.

10. Composés selon la revendication 1, caractérisés par le fait que le radical aryle est un radical phényle éventuellement substitué par au moins un atome d'halogène, un hydroxyle ou une fonction nitro.

11. Composés selon la revendication 1, caractérisés par le fait que le radical aralkyle est le radical benzyle ou phénéthyle éventuellement substitué par au moins un atome d'halogène, un hydroxyle ou une fonction nitro.

12. Composés selon la revendication 1, caractérisés par le fait que le radical alkynyle ayant de 2 à 6 atomes de carbone est le radical propargyle.

13. Composés selon la revendication 1, caractérisés par le fait que le radical alkényle ayant de 2 à 6 atomes de carbone est pris dans le groupe constitué par le radical vinyle, propényle, 2-méthyl propényle et butène-2-yle.

14. Composés selon la revendication 1, caractérisés par le fait que le radical fluoroalkyle inférieur est un radical ayant de 1 à 6 atomes de carbone et de 3 à 7 atomes de fluor.

15. Composés selon la revendication 1, caractérisés par le fait que l'hétérocycle est pris dans le groupe constitué par un radical pipéridino, morpholino, pyrrolidino ou pipérazino, éventuellement substitué en position 4 par un radical alkyle en C₁-C₆ ou mono ou polyhydroxyalkyle.

16. Composés selon l'une quelconque des revendications précédentes, caractérisés par le fait qu'ils sont pris dans le groupe constitué par :
1) Acide 4-(N-méthyl-5,6,7,8-tétrahydro-5,5,8,8-tétraméthyl-2-naphtylcarboxamidino) benzoïque,
2) Acide 4-(5,6,7,8-tétrahydro-5,5,8,8-tétraméthyl-2-naphtylcarboxamidino) benzoïque,
3) Acide 4-[5-(1-adamantyl)-2 hydroxy-4-méthoxybenzylidèneamino]benzoïque,
4) Acide 4-[3-(1-adamantyl)-4-méthoxybenzamidino]benzoïque,
5) Chlorhydrate de l'acide 4-[N-méthyl-3-(1-adamantyl)-4-methoxybenzamidino]benzoïque,
6) Acide 4-[3-(1-adamantyl)-4-méthoxyphénylformamidinométhyl thioéther]benzoïque,
7) Acide 4-[3-(1-adamantyl)-4-méthoxyphénylformamidinométhyléther]benzoïque,
8) 4-[3-(1-adamantyl)-4-méthoxybenzylidèneamino]benzoate de méthyle,
9) Acide 4-[3-(1-adamantyl)-4-méthoxybenzylidèneamino]benzoïque,
10) Acide 4-(3-tert-butyl-4-méthoxybenzamidino)benzoïque
11) 4-(5,6,7,8-tétrahydro-5,5,8,8-tétraméthyl-2- naphtylcarboxamidino)benzoate de métthyle
12) Alcool 4-(5,6,7,8-tétrahydro-5,5,8,8-tétraméthyl-2- naphtylcarboxamidino)benzylique
13) 4-(5,6,7,8-tétrahydro-5,5,8,8-tétraméthyl-2- naphtylcarboxamidino)toluène
14) 4-(5,6,7,8-tétrahydro-5,5,8,8-tétraméthyl-2- naphtylcarboxamidino)benzamide
15) Acide 2-hydroxy-4-(5,6,7,8-tétrahydro-5,5,8,8-tétraméthyl-2- naphtylcarboxamidino)benzoïque
16) 4-(α-chloro-5,6,7,8-tétrahydro-5,5,8,8-tétraméthyl-2- naphtylméthylimino)benzoate d'allyle
17) Acide 4-(N¹-phényl-5,6,7,8-tétrahydro-5,5,8,8-tétraméthyl-2-naphtylcarboxamidino)benzoïque
18) Chlorhydrate de l'acide 4-(N¹-phényl-5,6,7,8-tétrahydro-5,5,8,8-tétraméthyl-2-naphtylcarboxamidino)benzoïque
19) Acide 4-(N¹-benzyl-5,6,7,8-tétrahydro-5,5,8,8-tétraméthyl-2-naphtyl -carboxamidino)benzoïque
20) Acide 4-(N¹,N¹-diméthyl-5,6,7,8-tétrahydro-5,5,8,8-tétraméthyl-2-naphtyl -carboxamidino)benzoïque
21) Acide 4-[N²-(5,6,7,8-tétrahydro-5,5,8,8-tétraméthyl-2- naphtyl)amidino]benzoïque
22) Acide 4-[N¹-phényl-N2-(5,6,7,8-tétrahydro-5,5,8,8-tétraméthyl- 2-naphtyl)amidino]benzoïque
23) Chlorhydrate du 4-(5,6,7,8-tétrahydro-5,5,8,8-tétraméthyl-2- naphtylcarboxamidino) phénol
24) Acide 4-(N¹-méthyl-N²-méthyl-5,6,7,8-tétrahydro-5,5,8,8-tétraméthyl-2-naphtylcarboxamidino)benzoïque
25) Acide 4-[N-(3,3,3-trifluoéthyl)-5,6,7,8-tétrahydro-5,5,8,8-tétraméthyl-2-naphtylcarboxamidino]benzoïque.

17. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 16.

18. Composition selon la revendication 17, caractérisée par le fait qu'elle contient de 0,001 à environ 5% en poids d'un composé de formule (I).

19. Utilisation d'un composé selon l'une quelconque des revendications 1 à 16 pour la préparation d'une composition pharmaceutique destinée au traitement des affections dermatologiques, rhumatismales, respiratoires ainsi qu'ophtalmologiques.

20. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 16.

21. Composition cosmétique selon la revendication 20, caractérisée par le fait qu'elle contient le composé de formule (I) à une concentration comprise entre 0,001 et 3%.

## Revendications (Revendications pour l'(les) Etat(s) contractant(s) suivant(s): ES)

1. Composition cosmétique pour l'hygiène corporelle et capillaire, caractérisée par le fait qu'elle contient, dans un véhicule cosmétique approprié, au moins un composé de formule (I) : 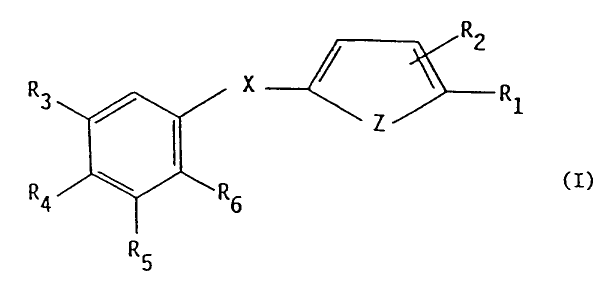 dans laquelle :
R₁ représente un atome d'hydrogène, le groupe OH, le radical -CH₃, le radical -CH₂OH, le radical -COR₇, le radical -CH(OH)CH₃, le radical -CH₂OCOR₈, le radical -SO₂R₉, le radical SOR₉ ou le radical -SR₉,
R₇ représentant un atome d'hydrogène, le groupe OH, le radical -OR₁₀, le radical : 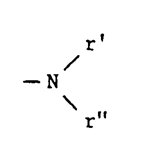
un radical alkyle inférieur, un radical monohydroxyalkyle, un radical polyhydroxyalkyle ou le reste d'un sucre,
R₈ représentant un radical alkyle linéaire ou ramifié, ayant de 1 à 20 atomes de carbone, un radical alkényle ayant de 2 à 20 atomes de carbone ou le reste d'un sucre,
R₉ représentant le groupe OH, un radical alkyle inférieur ou le radical 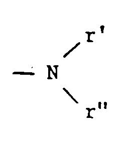 R₁₀ représentant un radical alkyle ayant de 1 à 20 atomes de carbone ou un radical alkényle ayant de 2 à 20 atomes de carbone,
r' et r'', identiques ou différents, représentent un atome d'hydrogène, un radical alkyle inférieur, un radical aryle, un radical aralkyle, un reste d'aminoacide, un reste de sucre, un reste de sucre aminé ou un hétérocyle ou r' et r'' pris ensemble forment un hétérocycle,
R₂ et R₆ représentent un atome d'hydrogène, le groupe OH, un radical alkyle inférieur, un radical alkoxy ayant de 1 à 6 atomes de carbone, un atome de fluor, un atome de chlore ou le groupe CF₃,
R₃ et R₅ représentent un radical alkyle α,α'-disubstitué ayant de 4 à 12 atomes de carbone ou un radical cycloalkyle mono ou polycyclique ayant de 5 à 12 atomes de carbone dont le carbone de liaison est trisubstitué,
R₄ représente un atome d'hydrogène, le groupe OH, un radical alkoxy ayant de 1 à 6 atomes de carbone, un radical alkyle α,α'-disubstitué ayant de 4 à 12 atomes de carbone,
R₃ et R₄ ou R₄ et R₅ pris ensemble peuvent former, avec le noyau benzénique adjacent, un cycle à 5 ou 6 atomes de carbone substitués par 2 à 6 groupes méthyles,
Z représente le radical divalent -CH=CR₁₁-
R₁₁ représentant un atome d'hydrogène, le groupe OH ou un radical alkyle inférieur,
X est choisi parmi les radicaux :
(i) - CR₁₃= N -
(ii) - N = CR₁₃-
(iii) 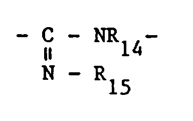
(iv) 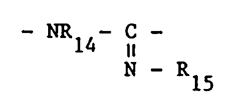
R₁₃ représentant le radical R₁₆, le radical OR₁₆, le radical -SR₁₆ ou le radical : 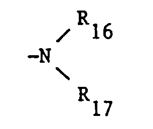 R₁₆ et R₁₇ représentant un atome d'hydrogène, un radical alkyle inférieur, un radical fluoroalkyle inférieur, un radical alkényle ayant de 2 à 6 atomes de carbone, un radical alkynyle ayant de 2 à 6 atomes de carbone, un radical aryle ou un radical aralkyle,
R₁₄ représentant un radical alkyle inférieur,
R₁₅ représentant un radical alkyle inférieur ou un radical fluoroalkyle inférieur,
à l'exclusion des composés de Formule (I) dans laquelle R₃ et R₅ sont identiques lorsque X représente le radical - CR₁₃= N - , R₁₃ représentant un atome d'hydrogène,
et les sels desdits composés de formule (I) obtenus par addition de base lorsque R₁ représente une fonction acide carboxylique ou par addition d'acide.

2. Composition cosmétique selon la revendication 1, caractérisée par le fait qu'elle contient le composé de formule (I) à une concentration comprise entre 0,001 et 3 %.

3. Composition selon la revendication 1, caractérisée par le fait que le composé de formule (I) se présente sous forme de sels d'un métal alcalin ou alcalino-terreux ou encore de zinc ou d'une amine organique.

4. Composition selon la revendication 1, caractérisée par le fait que le composé de formule (I) se présente sous forme de sels d'un acide minéral ou organique choisi parmi l'acide chlorhydrique, sulfurique, acétique, citrique, fumarique, hémisuccinique, maléique et mandélique.

5. Composition selon l'une quelconque des revendications précédentes, caractérisée par le fait que le composé de formule (I) est pris dans le groupe constitué par :
1) Acide 4-(N-méthyl-5,6,7,8-tétrahydro-5,5,8,8-tétraméthyl-2-naphtylcarboxamidino)benzoïque,
2) Acide 4-(5,6,7,8-tétrahydro-5,5,8,8-tétraméthyl-2-naphtylcarboxamidino)benzoïque,
3) Acide 4-[5-(1-adamantyl)-2 hydroxy-4-méthoxybenzylidèneamino] benzoïque,
4) Acide 4-[3-(1-adamantyl)-4-méthoxybenzamidino]benzoïque,
5) Chlorhydrate de l'acide 4-[N-méthyl-3-(1-adamantyl)-4-methoxybenzamidino]benzoïque,
6) Acide 4-[3-(1-adamantyl)-4-méthoxyphénylformamidinométhyl thioéther]benzoïque,
7) Acide 4-[3-(1-adamantyl)-4-méthoxyphénylformamidinométhyléther] benzoïque,
8) 4-[3-(1-adamantyl)-4-méthoxybenzylidèneamino]benzoate de méthyle,
9) Acide 4-[3-(1-adamantyl)-4-méthoxybenzylidèneamino]benzoïque,
10) Acide 4-(3-tert-butyl-4-méthoxybenzamidino)benzoïque
11) 4-(5,6,7,8-tétrahydro-5,5,8,8-tétraméthyl-2- naphtylcarboxamidino)benzoate de méthyle
12) Alcool 4-(5,6,7,8-tétrahydro-5,5,8,8-tétraméthyl-2- naphtylcarboxamidino)benzylique
13) 4-(5,6,7,8-tétrahydro-5,5,8,8-tétraméthyl-2- naphtylcarboxamidino)toluène
14) 4-(5,6,7,8-tétrahydro-5,5,8,8-tétraméthyl-2- naphtylcarboxamidino)benzamide
15) Acide 2-hydroxy-4-(5,6,7,8-tétrahydro-5,5,8,8-tétraméthyl-2- naphtylcarboxamidino) benzoïque
16) 4-(α-chloro-5,6,7,8-tétrahydro-5,5,8,8-tétraméthyl-2- naphtylméthylimino)benzoate d'allyle
17) Acide 4-(N¹-phényl-5,6,7,8-tétrahydro-5,5,8,8-tétraméthyl-2-naphtylcarboxamidino)benzoïque
18) Chlorhydrate de l'acide 4-(N¹-phényl-5,6,7,8-tétrahydro-5,5,8,8-tétraméthyl-2-naphtylcarboxamidino)benzoïque
19) Acide 4-(N¹-benzyl-5,6,7,8-tétrahydro-5,5,8,8-tétraméthyl-2-naphtylcarboxamidino)benzoïque
20) Acide 4-(N¹,N¹-diméthyl-5,6,7,8-tétrahydro-5,5,8,8-tétraméthyl-2-naphtylcarboxamidino)benzoïque
21) Acide 4-[N²-(5,6,7,8-tétrahydro-5,5,8,8-tétraméthyl-2- naphtyl)amidino]benzoïque
22) Acide 4-[N¹-phényl-N2-(5,6,7,8-tétrahydro-5,5,8,8-tétraméthyl- 2-naphtyl)amidino] benzoïque
23) Chlorhydrate du 4-(5,6,7,8-tétrahydro-5,5,8,8-tétraméthyl-2- naphtylcarboxamidino) phénol
24) Acide 4-(N¹-méthyl-N²-méthyl-5,6,7,8-tétrahydro-5,5,8,8-tétraméthyl-2-naphtylcarboxamidino)benzoïque
25) Acide 4-[N-(3,3,3-trifluoroéthyl)-5,6,7,8-tétrahydro-5,5,8,8-tétraméthyl-2-naphtylcarboxamidino]benzoïque.

## Claims (Claims for the following Contracting State(s): AT, BE, CH, DE, DK, FR, GB, GR, IT, LI, NL, PT, SE)

1. Biaromatic compounds, characterized in that they correspond to the following general formula: 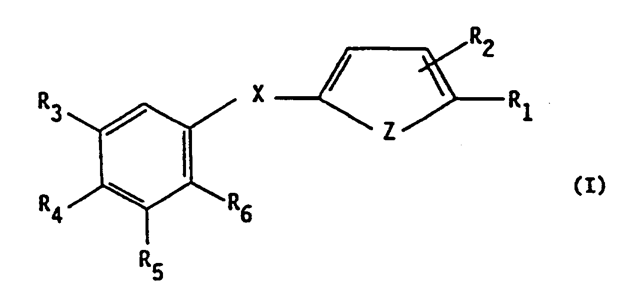 in which:
R₁ represents a hydrogen atom, the OH group, the -CH₃ radical, the -CH₂OH radical, the -COR₇ radical, the -CH(OH)CH₃ radical, the -CH₂OCOR₈ radical, the -SO₂R₉ radical, the SOR₉ radical or the -SR₉ radical,
R₇ representing a hydrogen atom, the OH group, the -OR₁₀ radical, the radical: 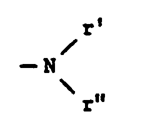 a lower alkyl radical, a monohydroxyalkyl radical, a polyhydroxyalkyl radical or the residue of a sugar ,
R⁸ representing a linear or branched alkyl radical having from 1 to 20 carbon atoms, an alkenyl radical having from 2 to 20 carbon atoms or the residue of a sugar,
R₉ representing the OH group, a lower alkyl radical or the radical 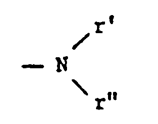 R₁₀ representing an alkyl radical having from 1 to 20 carbon atoms or an alkenyl radical having from 2 to 20 carbon atoms,
r' and r'', which are identical or different, represent a hydrogen atom, a lower alkyl radical, an aryl radical, an aralkyl radical, an amino acid residue, a sugar residue, an amino sugar residue or a heterocycle or r' and r'', taken together, form a heterocycle,
R₂ and R₆ represent a hydrogen atom, the OH group, a lower alkyl radical, an alkoxy radical having from 1 to 6 carbon atoms, a fluorine atom, a chlorine atom or the CF₃ group,
R₃ and R₅ represent an α,α'-disubstituted alkyl radical having from 4 to 12 carbon atoms or a mono- or polycyclic cycloalkyl radical having from 5 to 12 carbon atoms in which the bonding carbon is trisubstituted,
R₄ represents a hydrogen atom, the OH group, an alkoxy radical having from 1 to 6 carbon atoms, or an α,α'-disubstituted alkyl radical having from 4 to 12 carbon atoms,
R₃ and R₄ or R₄ and R₅, taken together, can form, with the adjacent benzene ring, a ring containing 5 or 6 carbon atoms substituted by 2 to 6 methyl groups,
Z represents the divalent -CH=CR₁₁- radical,
R₁₁ representing a hydrogen atom, the OH group or a lower alkyl radical,
X is chosen from the radicals:
(i) -CR₁₃=N-
(ii) -N=CR₁₃-
(iii) 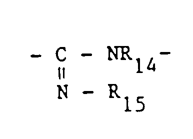
(iv) 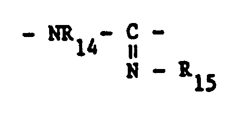
R₁₃ representing the R₁₆ radical, the OR₁₆ radical, the -SR₁₆ radical or the radical: 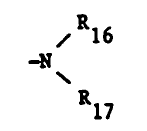 R₁₆ and R₁₇ representing a hydrogen atom, a lower alkyl radical, a lower fluoroalkyl radical, an alkenyl radical having from 2 to 6 carbon atoms, an alkynyl radical having from 2 to 6 carbon atoms, an aryl radical or an aralkyl radical,
R₁₄ representing a lower alkyl radical,
R₁₅ representing a lower alkyl radical or a lower fluoroalkyl radical,
with the exception of the compounds of formula (I) in which R₃ and R₅ are identical when X represents the -CR₁₃=N- radical, R₁₃ representing a hydrogen atom,
and the salts of the said compounds of formula (I) obtained by addition of base when R₁ represents a carboxylic acid functional group or by addition of acid.

2. Compounds according to Claim 1, characterized in that they are provided in the form of salts of an alkali metal or alkaline-earth metal or alternatively of zinc or of an organic amine.

3. Compounds according to Claim 1, characterized in that they are provided in the form of salts of an inorganic or organic acid chosen from hydrochloric, sulphuric, acetic, citric, fumaric, hemisuccinic, maleic and mandelic acid.

4. Compounds according to Claim 1, characterized in that the lower alkyl radical has from 1 to 6 carbon atoms and is taken from the group consisting of the methyl, ethyl, isopropyl, butyl and tert-butyl radicals.

5. Compounds according to Claim 1, characterized in that the alkoxy radical having from 1 to 6 carbon atoms is taken from the group consisting of a methoxy, ethoxy, isopropoxy and butoxy radical.

6. Compounds according to Claim 1, characterized in that the α,α'-disubstituted alkyl radical is taken from the group consisting of: a tert-butyl, 1,1-dimethylpropyl, 1-methyl-1-ethylpropyl, 1-methyl-1-ethylhexyl and 1,1-dimethyldecyl radical.

7. Compounds according to Claim 1, characterized in that the mono- or polycyclic cycloalkyl radical having from 5 to 12 carbon atoms in which the bonding carbon is trisubstituted is the 1-methylcyclohexyl or 1-adamantyl radical.

8. Compounds according to Claim 1, characterized in that the monohydroxyalkyl radical is a 2-hydroxyethyl, 2-hydroxypropyl or 3-hydroxypropyl radical.

9. Compounds according to Claim 1, characterized in that the polyhydroxyalkyl radical contains from 2 to 6 carbon atoms and from 2 to 5 hydroxyl groups and is taken from the group consisting of the 2,3-dihydroxypropyl, 2,3,4-trihydroxybutyl and 2,3,4,5-tetrahydroxypentyl radical and the pentaerythritol residue.

10. Compounds according to Claim 1, characterized in that the aryl radical is a phenyl radical optionally substituted by at least one halogen atom, one hydroxyl or one nitro functional group.

11. Compounds according to Claim 1, characterized in that the aralkyl radical is the benzyl or phenethyl radical optionally substituted by at least one halogen atom, one hydroxyl or one nitro functional group.

12. Compounds according to Claim 1, characterized in that the alkynyl radical having from 2 to 6 carbon atoms is the propargyl radical.

13. Compounds according to Claim 1, characterized in that the alkenyl radical having from 2 to 6 carbon atoms is taken from the group consisting of the vinyl, propenyl, 2-methylpropenyl and buten-2-yl radical.

14. Compounds according to Claim 1, characterized in that the lower fluoroalkyl radical is a radical having from 1 to 6 carbon atoms and from 3 to 7 fluorine atoms.

15. Compounds according to Claim 1, characterized in that the heterocycle is taken from the group comprising a piperidino, morpholino, pyrrolidino or piperazino radical, optionally substituted in the 4-position by a mono- or polyhydroxyalkyl or C₁-C₆ alkyl radical.

16. Compounds according to any one of the preceding claims, characterized in that they are taken from the group consisting of:
1) 4-(N-Methyl-5,6,7,8-tetrahydro-5,5,8,8-tetramethyl-2-naphthylcarboxamidino)benzoic acid
2) 4-(5,6,7,8-Tetrahydro-5,5,8,8-tetramethyl-2-naphthylcarboxamidino)benzoic acid
3) 4-[5-(1-Adamantyl)-2-hydroxy-4-methoxybenzylideneamino]benzoic acid
4) 4-[3-(1-Adamantyl)-4-methoxybenzamidino]benzoic acid
5) 4-[N-Methyl-3-(1-adamantyl)-4-methoxybenzamidino]benzoic acid hydrochloride
6) 4-[α-Methylthio-3-(1-adamantyl)-4-methoxyphenylmethylimino]benzoic acid
7) 4-[α-Methoxy-3-(1-adamantyl)-4-methoxyphenylmethylimino]benzoic acid
8) Methyl 4-[3-(1-adamantyl)-4-methoxybenzylideneamino]benzoate
9) 4-[3-(1-Adamantyl)-4-methoxybenzylideneamino]benzoic acid
10) 4-(3-tert-Butyl-4-methoxybenzamidino)benzoic acid
11) Methyl 4-(5,6,7,8-tetrahydro-5,5,8,8-tetramethyl-2-naphthylcarboxamidino)benzoate
12) 4-(5,6,7,8-Tetrahydro-5,5,8,8-tetramethyl-2-naphthylcarboxamidino)benzyl alcohol
13) 4-(5,6,7,8-Tetrahydro-5,5,8,8-tetramethyl-2-naphthylcarboxamidino)toluene
14) 4-(5,6,7,8-Tetrahydro-5,5,8,8-tetramethyl-2-naphthylcarboxamidino)benzamide
15) 2-Hydroxy-4-(5,6,7,8-tetrahydro-5,5,8,8-tetramethyl-2-naphthylcarboxamidino)benzoic acid
16) Allyl 4-(α-chloro-5,6,7,8-tetrahydro-5,5,8,8-tetramethyl-2-napthylmethylimino)benzoate
17) 4-(N¹-Phenyl-5,6,7,8-tetrahydro-5,5,8,8-tetramethyl-2-naphthylcarboxamidino)benzoic acid
18) 4-(N¹-Phenyl-5,6,7,8-tetrahydro-5,5,8,8-tetramethyl-2-naphthylcarboxamidino)benzoic acid hydrochloride
19) 4-(N¹-Benzyl-5,6,7,8-tetrahydro-5,5,8,8-tetramethyl-2-naphthylcarboxamidino)benzoic acid
20) 4-(N¹,N¹-Dimethyl-5,6,7,8-tetrahydro-5,5,8,8-tetramethyl-2-naphthylcarboxamidino)benzoic acid
21) 4-[N²-(5,6,7,8-Tetrahydro-5,5,8,8-tetramethyl-2-naphthyl)amidino]benzoic acid
22) 4-[N¹-Phenyl-N²-(5,6,7,8-tetrahydro-5,5,8,8-tetramethyl-2-naphthyl)amidino]benzoic acid
23) 4-(5,6,7,8-Tetrahydro-5,5,8,8-tetramethyl-2-naphthylcarboxamidino)phenol hydrochloride
24) 4-(N¹-Methyl-N²-methyl-5,6,7,8-tetrahydro-5,5,8,8-tetramethyl-2-naphthylcarboxamidino)benzoic acid
25) 4-[N-(3,3,3-Trifluoroethyl)-5,6,7,8-tetrahydro-5,5,8,8-tetramethyl-2-naphthylcarboxamidino]benzoic acid.

17. Pharmaceutical composition, characterized in that it contains, in an appropriate vehicle for enteral, parenteral, topical or ocular administration, at least one compound of formula (I) according to any one of Claims 1 to 16.

18. Composition according to Claim 17, characterized in that it contains from 0.001 to approximately 5 % by weight of a compound of formula (I).

19. Use of a compound according to any one of Claims 1 to 16 for the preparation of a pharmaceutical composition intended for the treatment of dermatological, rheumatic, respiratory and ophthalmological conditions.

20. Cosmetic composition for body and hair hygiene, characterized in that it contains, in an appropriate cosmetic vehicle, at least one compound of formula (I) according to any one of Claims 1 to 16.

21. Cosmetic composition according to Claim 20, characterized in that it contains the compound of formula (I) at a concentration of between 0.001 and 3 %.

## Claims (Claims for the following Contracting State(s): ES)

1. Cosmetic composition for body and hair hygiene, characterized in that it contains, in an appropriate cosmetic vehicle, at least one compound of formula (I): 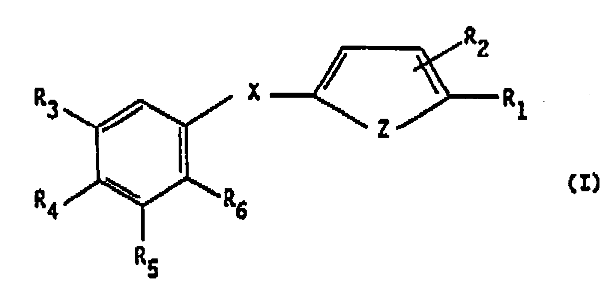 in which:
R₁ represents a hydrogen atom, the OH group, the -CH₃ radical, the -CH₂OH radical, the -COR₇ radical, the -CH(OH)CH₃ radical, the -CH₂OCOR₈ radical, the -SO₂R₉ radical, the SOR₉ radical or the -SR₉ radical,
R₇ representing a hydrogen atom, the OH group, the -OR₁₀ radical, the radical: 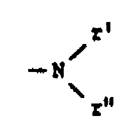 a lower alkyl radical, a monohydroxyalkyl radical, a polyhydroxyalkyl radical or the residue of a sugar ,
R₈ representing a linear or branched alkyl radical having from 1 to 20 carbon atoms, an alkenyl radical having from 2 to 20 carbon atoms or the residue of a sugar,
R₉ representing the OH group, a lower alkyl radical or the radical 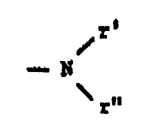 R₁₀ representing an alkyl radical having from 1 to 20 carbon atoms or an alkenyl radical having from 2 to 20 carbon atoms,
r' and r'', which are identical or differant, represent a hydrogen atom, a lower alkyl radical, an aryl radical, an aralkyl radical, an amino acid residue, a sugar residue, an amino sugar residue or a heterocycle or r' and r'', taken together, form a heterocycle,
R₂ and R₆ represent a hydrogen atom, the OH group, a lower alkyl radical, an alkoxy radical having from 1 to 6 carbon atoms, a fluorine atom, a chlorine atom or the CF₃ group,
R₃ and R₅ represent an α,α'-disubstituted alkyl radical having from 4 to 12 carbon atoms or a mono- or polycyclic cycloalkyl radical having from 5 to 12 carbon atoms in which the bonding carbon is trisubstituted,
R₄ represents a hydrogen atom, the OH group, an alkoxy radical having from 1 to 6 carbon atoms, or an α,α'-disubstituted alkyl radical having from 4 to 12 carbon atoms,
R₃ and R₄ or R₄ and R₅, taken together, can form, with the adjacent benzene ring, a ring containing 5 or 6 carbon atoms substituted by 2 to 6 methyl groups,
Z represents the divalent -CH=CR₁₁- radical,
R₁₁ representing a hydrogen atom, the OH group or a lower alkyl radical,
X is chosen from the radicals:
(i) -CR₁₃=N-
(ii) -N=CR₁₃-
(iii) 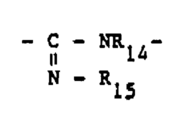
(iv) 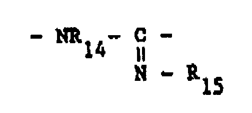
R₁₃ representing the R₁₆ radical, the OR₁₆ radical, the -SR₁₆ radical or the radical: 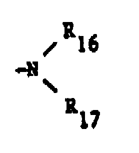 R₁₆ and R₁₇ representing a hydrogen atom, a lower alkyl radical, a lower fluoroalkyl radical, an alkenyl radical having from 2 to 6 carbon atoms, an alkynyl radical having from 2 to 6 carbon atoms, an aryl radical or an aralkyl radical,
R₁₄ representing a lower alkyl radical,
R₁₅ representing a lower alkyl radical or a lower fluoroalkyl radical,
with the exception of the compounds of formula (I) in which R₂ and R₅ are identical when X represents the -CR₁₃=N- radical, R₁₃ representing a hydrogen atom,
and the salts of the said compounds of formula (I) obtained by addition of base when R₁ represents a carboxylic acid functional group or by addition of acid.

2. Cosmetic composition according to Claim 1, characterized in that it contains the compound of formula (I) at a concentration of between 0.001 and 3 %.

3. Composition according to Claim 1, characterized in that the compound of formula (I) is provided in the form of salts of an alkali metal or alkaline-earth metal or alternatively of zinc or of an organic amine.

4. Composition according to Claim 1, characterized in that the compound of formula (I) is provided in the form of salts of an inorganic or organic acid chosen from hydrochloric, sulphuric, acetic, citric, fumaric, hemisuccinic, maleic and mandelic acid,

5. Composition according to any one of the preceding claims, characterized in that the compound of formula (I) is taken from the group consisting of:
1) 4-(N-Methyl-5,6,7,8-tetrahydro-5,5,8,8-tetramethyl-2-naphthylcarboxamidino)benzoic acid
2) 4-(5,6,7,8-Tetrahydro-5,5,8,8-tetramethyl-2-naphthylcarboxamidino)benzoic acid
3) 4-[5-(1-Adamantyl)-2-hydroxy-4-methoxybenzylideneamino]benzoic acid
4) 4-[3-(1-Adamantyl)-4-methoxybenzamidino]benzoic acid
5) 4-[N-Methyl-3-(1-adamantyl)-4-methoxybenzamidino]benzoic acid hydrochloride
6) 4-[α-Methylthio-3-(1-adamantyl)-4-methoxyphenylmethylimino]benzoic acid
7) 4-[α-Methoxy-3-(1-adamantyl)-4-methoxyphenylmethylimino]benzoic acid
8) Methyl 4-[3-(1-adamantyl)-4-methoxybenzylideneamino]benzoate
9) 4-[3-(1-Adamantyl)-4-methoxybenzylideneamino]benzoic acid
10) 4-(3-tert-Butyl-4-methoxybenzamidino)benzoic acid
11) Methyl-4-(5,6,7,8-tetrahydro-5,5,8,8-tetramethyl-2-naphthylcarboxamidino)benzoate
12) 4-(5,6,7,8-Tetrahydro-5,5,8,8-tetramethyl-2-naphthylcarboxamidino)benzyl alcohol
13) 4-(5,6,7,8-Tetrahydro-5,5,8,8-tetramethyl-2-naphthylcarboxamidino)toluene
14) 4-(5,6,7,8-Tetrahydro-5,5,8,8-tetramethyl-2-naphthylcarboxamidino)benzamide
15) 2-Hydroxy-4-(5,6,7,8-tetrahydro-5,5,8,8-tetramethyl-2-naphthylcarboxamidino)benzoic acid
16) Allyl 4-(α-chloro-5,6,7,8-tetrahydro-5,5,8,8-tetramethyl-2-napthylmethylimino)benzoate
17) 4-(N¹-Phenyl-5,6,7,8-tetrahydro-5,5,8,8-tetramethyl-2-naphthylcarboxamidino)benzoic acid
18) 4-(N¹-Phenyl-5,6,7,8-tetrahydro-5,5,8,8-tetramethyl-2-naphthylcarboxamidino)benzoic acid hydrochloride
19) 4-(N¹-Benzyl-5,6,7,8-tetrahydro-5,5,8,8-tetramethyl-2-naphthylcarboxamidino)benzoic acid
20) 4-(N¹,N¹-Dimethyl-5,6,7,8-tetrahydro-5,5,8,8-tetramethyl-2-naphthylcarboxamidino)benzoic acid
21) 4-[N²-(5,6,7,8-Tetrahydro-5,5,8,8-tetramethyl-2-naphthyl)amidino]benzoic acid
22) 4-[N¹-Phenyl-N²-(5,6,7,8-tetrahydro-5,5,8,8-tetramethyl-2-naphthyl)amidino]benzoic acid
23) 4-(5,6,7,8-Tetrahydro-5,5,8,8-tetramethyl-2-naphthylcarboxamidino)phenol hydrochloride
24) 4-(N¹-Methyl-N²-methyl-5,6,7,8-tetrahydro-5,5,8,8-tetramethyl-2-naphthylcarboxamidino)benzoic acid
25) 4-[N-(3,3,3-Trifluoroethyl)-5,6,7,8-tetrahydro-5,5,8,8-tetramethyl-2-naphthylcarboxamidino]benzoic acid.

## Patentansprüche (Patentansprüche für folgende(n) Vertragsstaat(en): AT, BE, CH, DE, DK, FR, GB, GR, IT, LI, NL, PT, SE)

1. Diaromatische Verbindungen, dadurch gekennzeichnet, daß sie die folgende allgemeine Formel aufweisen: 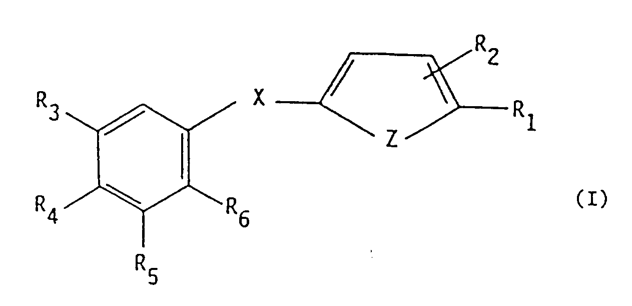 worin
R₁ ein Wasserstoffatom, die OH-Gruppe, die -CH₃-Gruppe, die -CH₂OH-Gruppe, den Rest -COR₇, den Rest -CH(OH)CH₃, den Rest -CH₂OCOR₈, den Rest -SO₂R₉, den Rest SOR₉ oder den Rest -SR₉ bedeutet,
R₇ ein Wasserstoffatom, die OH-Gruppe, die -OR₁₀-Gruppe, den Rest 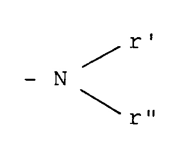 einen niedrigen Alkylrest, einen Monohydroxyalkylrest, einen Polyhydroxyalkylrest oder einen Zuckerrest bedeutet,
R₈ einen linearen oder verzweigten Alkylrest mit 1 bis 20 Kohlenstoffatomen, einen Alkenylrest mit 2 bis 20 Kohlenstoffatomen oder einen Zuckerrest darstellt,
R₉ die OH-Gruppe, einen niedrigen Alkylrest oder den Rest 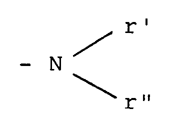 bedeutet, wobei R₁₀ einen Alkylrest mit 1 bis 20 Kohlenstoffatomen oder einen Alkenylrest mit 2 bis 20 Kohlenstoffatomen bedeutet und
r' und r'', die gleich oder verschieden sind, ein Wasserstoffatom, einen niedrigen Alkylrest, einen Arylrest, einen Aminosäurerest, einen Zuckerrest, einen aminierten Zucker- oder einen Heterocyclus darstellen oder r' und r'' zusammengenommen einen Heterocyclus bilden,
R₂ und R₆ ein Wasserstoffatom, die OH-Gruppe, einen niedrigen Alkylrest, einen Alkoxyrest mit 1 bis 6 Kohlenstoffatomen, ein Fluoratom, ein Chloratom oder die CF₃-Gruppe darstellen,
R₃ und R₅ einen α,α'-disubstituierten Alkylrest mit 4 bis 12 Kohlenstoffatomen oder einen mono- oder polyzyklischen Cycloalkylrest mit 5 bis 12 Kohlenstoffatomen bedeuten, bei dem das Bindungskohlenstoffatom dreifach substituiert ist,
R₄ ein Wasserstoffatom, die OH-Gruppe, einen Alkoxyrest mit 1 bis 6 Kohlenstoffatomen, einen α,α'-disubstituierten Alkylrest mit 4 bis 12 Kohlenstoffatomen darstellt,
R₃ und R₄ oder R₄ und R₅ zusammen mit dem benachbarten Benzolrest einen Ring mit 5 oder 6 Kohlenstoffatomen bilden, welche jeweils durch 2 bis 6 Methylgruppen substituiert sind,
Z den zweiwertigen -CH=CR₁₁ -Rest,
R₁₁ ein Wasserstoffatom, die OH-Gruppe oder einen niedrigen Alkylrest darstellt,
X aus den folgenden Resten ausgewählt ist:
(i) - CR₁₃ = N -
(ii) - N = CR₁₃ -
(iii) 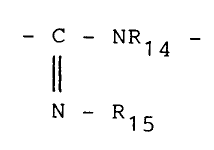
(iv) 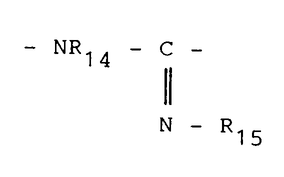
R₁₃ den Rest R₁₆, den Rest OR₁₆, den Rest -SR₁₆ oder den Rest 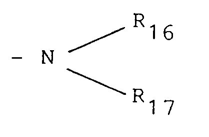 darstellt, wobei R₁₆ und R₁₇ ein Wasserstoffatom, einen niedrigen Alkylrest, einen niedrigen Fluoralkylrest, einen Alkenylrest mit 2 bis 6 Kohlenstoffatomen, einen Alkinylrest mit 2 bis 6 Kohlenstoffatomen, einen Aryl- oder Aralkylrest bedeuten,
R₁₄ einen niedrigen Alkylrest,
R₁₅ einen niedrigen Alkylrest oder einen niedrigen Fluoralkylrest
mit der Ausnahme bedeuten, daß die Verbindungen gemäß Formel (I), in der R₃ und R₅ gleich sind, insofern X den Rest -CR₁₃=N- darstellt, R₁₃ ein Wasserstoffatom bedeutet,
sowie die Salze der Verbindungen gemäß Formel (I), welche durch die Zugabe von Alkali erhalten wurden, und zwar in dem Falle, daß R₁ eine Carbonsäurefunktion bedeutet oder jene durch Zugabe von Säure erhalten wurden.

2. Verbindungen gemäß Anspruch 1, dadurch gekennzeichnet, daß sie in Form von Alkali- oder Erdalkalimetallsalzen vorliegen oder auch in Form von Zinksalzen oder in Form eines organischen Amins.

3. Verbindungen gemäß Anspruch 1, dadurch gekennzeichnet, daß sie in Form von Mineralsäure- oder organischen Säuresalzen vorliegen, welche unter Salzsäure, Schwefelsäure, Essigsäure, Zitronensäure, Fumarsäure, Hemibernsteinsäure, Maleinsäure und Mandelsäure ausgewählt sind.

4. Verbindungen gemäß Anspruch 1, dadurch gekennzeichnet, daß der niedrige Alkylrest zwischen 1 und 6 Kohlenstoffatomen aufweist und aus der Gruppe bestehend aus der Methyl-, Ethyl-, Isopropyl-, Butyl- und tert-Butylgruppe ausgewählt ist.

5. Verbindungen gemäß Anspruch 1, dadurch gekennzeichnet, daß der Alkoxyrest mit 1 bis 6 Kohlenstoffatomen aus der aus der Methoxy-, Ethoxy, Isopropoxy- und Butoxygruppe bestehenden Gruppe ausgewählt ist.

6. Verbindungen gemäß Anspruch 1, dadurch gekennzeichnet, daß der α,α'-disubstituierte Alkylrest aus der aus der tert-Butyl, 1,1-Dimethylpropyl-, 1-Methyl-1-ethylpropyl-, 1-Methyl-1-ethylhexyl- und 1,1-Dimethyldecylgruppe bestehenden Gruppe ausgewählt ist.

7. Verbindungen gemäß Anspruch 1, dadurch gekennzeichnet, daß der mono- oder polycyclische Cycloalkylrest mit 5 bis 12 Kohlenstoffatomen, bei dem das Bindungskohlenstoffatom dreifach substituiert ist, die 1-Methylcyclohexyl- oder 1-Adamantylgruppe bedeutet.

8. Verbindungen gemäß Anspruch 1, dadurch gekennzeichnet, daß der Monohydroxyalkylrest eine 2-Hydroxyethyl, 2-Hydroxypropyl- oder 3-Hydroxypropylgruppe bedeutet.

9. Verbindungen gemäß Anspruch 1, dadurch gekennzeichnet, daß der Polyhydroxyalkylrest 2 bis 6 Kohlenstoffatome und 2 bis 5 Hydroxylgruppen aufweist und aus der aus der 2,3-Dihydroxypropyl-, 2,3,4-Trihydroxybutyl-, 2,3,4,5-Tetrahydroxypentyl- und der Pentaerythritgruppe bestehenden Gruppe ausgewählt ist.

10. Verbindungen gemäß Anspruch 1, dadurch gekennzeichnet, daß der Arylrest eine gegebenenfalls durch mindestens ein Halogenatom, eine Hydroxylgruppe oder eine Nitrofunktion substituierte Phenylgruppe darstellt.

11. Verbindungen gemäß Anspruch 1, dadurch gekennzeichnet, daß der Allylrest die Benzyl- oder gegebenenfalls durch mindestens ein Halogenatom, eine Hydroxylgruppe oder eine Nitrofunktion substituierte Phenethylgruppe darstellt.

12. Verbindungen gemäß Anspruch 1, dadurch gekennzeichnet, daß der Alkinylrest mit 2 bis 6 Kohlenstoffatomen die Propargylgruppe bedeutet.

13. Verbindungen gemäß Anspruch 1, dadurch gekennzeichnet, daß der Alkenylrest mit 2 bis 6 Kohlenstoffatomen aus der aus der Vinyl-, Propenyl-, 2-Methylpropenyl- und Buten-2-yl-gruppe bestehenden Gruppe ausgewählt ist.

14. Verbindungen gemäß Anspruch 1, dadurch gekennzeichnet, daß der niedrige Fluoralkylrest einen Rest mit 1 bis 6 Kohlenstoffatomen und 3 bis 7 Fluoratomen bedeutet.

15. Verbindungen gemäß Anspruch 1, dadurch gekennzeichnet daß der Heterocyclus aus der aus einem Piperidin-, Morpholin-, Pyrrolidin- und Piperazingruppe bestehenden Gruppe ausgewählt ist, welche jeweils gegebenenfalls in der 4-Stellung durch einen Alkylrest mit 1 bis 6 Kohlenstoffatomen oder einem Mono- bzw. Polyhydroxyalkylrest substituiert ist.

16. Verbindungen gemäß einem der voranstehenden Ansprüche, dadurch gekennzeichnet, daß sie aus der folgenden Gruppe von Verbindungen, bestehend aus
(1) 4-(N-Methyl-5,6,7,8-tetrahydro-5,5,8,8,-tetramethyl-2-naphthylcarboxamidin)-benzoesäure,
(2) 4-(5,6,7,8-Tetrahydro-5,5,8,8,-tetramethyl-2-naphthylcarboxamidin)-benzoesäure,
(3) 4-[5-(1-Adamantyl)-2-hydroxy-4-methoxybenzylidenamino]-benzoesäure,
(4) 4-[3-(1-Adamantyl)-4-methoxybenzamidin]-benzoesäure,
(5) 4-[N-Methyl-3-(1-adamantyl)-4-methoxybenzamidin]-benzoesäure-hydrochlorid,
(6) 4-[3-(1-Adamantyl)-4-methoxyphenylformamidinmethylthioether]-benzoesäure,
(7) 4-[3-(1-Adamantyl)-4-methoxyphenylformamidinmethylether]-benzoesäure,
(8) 4-[3-(1-Adamantyl)-4-methoxybenzylidenamino]-methylbenzoat,
(9) 4-[3-(1-Adamantyl)-4-methoxybenzylidenamino]-benzoesäure,
(10) 4-(3-tert-Butyl-4-methoxybenzamidin)-benzoesäure,
(11) 4-(5,6,7,8-Tetrahydro-5,5,8,8-tetramethyl-2-naphthylcarboxamidin)-benzoesäuremethylester,
(12) 4-(5,6,7,8-Tetrahydro-5,5,8,8-tetramethyl-2-naphthylcarboxamidin)-benzylalkohol,
(13) 4-(5,6,7,8-Tetrahydro-5,5,8,8-tetramethyl-2-naphthylcarboxamidin)-toluol,
(14) 4-(5,6,7,8-Tetrahydro-5,5,8,8-tetramethyl-2-naphthylcarboxamidin)-benzamid,
(15) 2-Hydroxy-4-(5,6,7,8-tetrahydro-5,5,8,8-tetramethyl-2-naphthylcarboxamidin)-benzoesäure,
(16) 4-(α-Chlor-5,6,7,8-tetrahydro-5,5,8,8-tetramethyl-2-naphthylmethylimino)-benzoesäureallylester,
(17) 4-(N¹-Phenyl-5,6,7,8-tetrahydro-5,5,8,8-tetramethyl-2-naphthylcarboxamidin)-benzoesäure,
(18) 4-(N¹-Phenyl-5,6,7,8-tetrahydro-5,5,8,8-tetramethyl-2-naphthylcarboxamidin)-benzoesäurehydrochlorid,
(19) 4-(N¹-Benzyl-5,6,7,8-tetrahydro-5,5,8,8-tetramethyl-2-naphthylcarboxamidin)-benzoesäure,
(20) 4-(N¹,N¹-Dimethyl-5,6,7,8-tetrahydro-5,5,8,8-tetramethyl-2-naphthylcarboxamidin)-benzoesäure,
(21) 4-[N²-(5,6,7,8-Tetrahydro-5,5,8,8-tetramethyl-2-naphthyl)-amidin]-benzoesäure,
(22) 4-[N¹-Phenyl-N²-(5,6,7,8-tetrahydro-5,5,8,8-tetramethyl-2-naphthyl)-amidin]-benzoesäure,
(23) 4-(5,6,7,8-Tetrahydro-5,5,8,8-tetramethyl-2-naphthylcarboxamidin)-phenolhydrochlorid,
(24) 4-(N¹-Methyl-N²-methyl-5,6,7,8-tetrahydro-5,5,8,8-tetramethyl-2-naphthylcarboxamidin)-benzoesäure,
(25) 4-[N-(3,3,3-Trifluorethyl)-5,6,7,8-tetrahydro-5,5,8,8-tetramethyl-2-naphthylcarboxamidin]-benzoesäure
ausgewählt sind.

17. Arzneimittel, dadurch gekennzeichnet, daß es in einem geeigneten Träger für die enterale, parenterale, topische oder ophthalmologische Verabreichung mindestens eine Verbindung der Formel (I) gemäß einem der Ansprüche 1 bis 16 enthält.

18. Zubereitung gemäß Anspruch 17, dadurch gekennzeichnet, daß sie 0,001 bis etwa 5 Gew-% einer Verbindung gemäß der Formel (I) enthält.

19. Verwendung einer Verbindung gemäß einem der Ansprüche 1 bis 16 zur Herstellung eines Arzneimittels, das zur Behandlung dermatologischer, rheumatischer, respiratorischer und ophthalmologischer Affektionen bestimmt ist.

20. Kosmetische Zubereitung für die Körper- und Haarhygiene, dadurch gekennzeichnet, daß sie in einem geeigneten kosmetischen Träger mindestens eine Verbindung gemäß der Formel (I) nach einem der Ansprüche 1 bis 16 enthält.

21. Kosmetische Zubereitung gemäß Anspruch 20, dadurch gekennzeichnet, daß sie eine Verbindung gemäß der Formel (I) in einer Konzentration zwischen 0,001 und 3 % enthält.

## Patentansprüche (Patentansprüche für folgende(n) Vertragsstaat(en): ES)

1. Kosmetische Zubereitung für die Körperhygiene und Haarpflege, dadurch gekennzeichnet, daß sie in einem geeigneten kosmetischen Träger mindestens eine Verbindung der Formel (I) enthält: 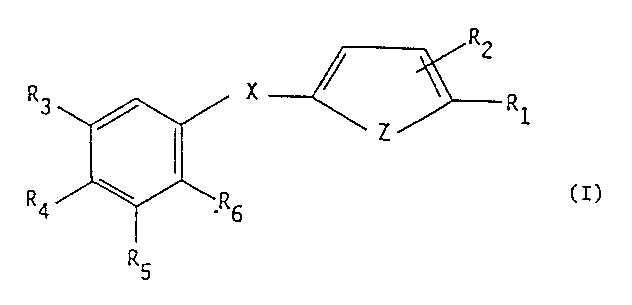 worin
R₁ ein Wasserstoffatom, die OH-Gruppe, die -CH₃-Gruppe, die -CH₂OH-Gruppe, den Rest -COR₇, den Rest -CH(OH)CH₃, den Rest -CH₂OCOR₈, den Rest -SO₂R₉, den Rest SOR₉ oder den Rest -SR₉ bedeutet,
R₇ ein Wasserstoffatom, die OH-Gruppe, die -OR₁₀-Gruppe, den Rest 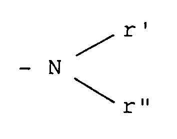 einen niedrigen Alkylrest, einen Monohydroxyalkylrest, einen Polyhydroxyalkylrest oder einen Zuckerrest bedeutet,
R₈ einen linearen oder verzweigten Alkylrest mit 1 bis 20 Kohlenstoffatomen, einen Alkenylrest mit 2 bis 20 Kohlenstoffatomen oder einen Zuckerrest darstellt,
R₉ die OH-Gruppe, einen niedrigen Alkylrest oder den Rest 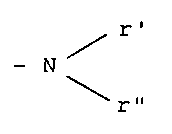 bedeutet, wobei R₁₀ einen Alkylrest mit 1 bis 20 Kohlenstoffatomen oder einen Alkenylrest mit 2 bis 20 Kohlenstoffatomen bedeutet und
r' und r'', die gleich oder verschieden sind, ein Wasserstoffatom, einen niedrigen Alkylrest, einen Arylrest, einen Aminosäurerest, einen Zuckerrest, einen aminierten Zucker- oder einen Heterocyclus darstellen oder r' und r'' zusammengenommen einen Heterocyclus bilden,
R₂ und R₆ ein Wasserstoffatom, die OH-Gruppe, einen niedrigen Alkylrest, einen Alkoxyrest mit 1 bis 6 Kohlenstoffatomen, ein Fluoratom, ein Chloratom oder die CF₃-Gruppe darstellen,
R₃ und R₅ einen α,α'-disubstituierten Alkylrest mit 4 bis 12 Kohlenstoffatomen oder einen mono- oder polyzyklischen Cycloalkylrest mit 5 bis 12 Kohlenstoffatomen bedeuten, bei dem das Bindungskohlenstoffatom dreifach substituiert ist,
R₄ ein Wasserstoffatom, die OH-Gruppe, einen Alkoxyrest mit 1 bis 6 Kohlenstoffatomen, einen α,α'-disubstituierten Alkylrest mit 4 bis 12 Kohlenstoffatomen darstellt,
R₃ und R₄ oder R₄ und R₅ zusammen mit dem benachbarten Benzolrest einen Ring mit 5 oder 6 Kohlenstoffatomen bilden, welche jeweils durch 2 bis 6 Methylgruppen substituiert sind,
Z den zweiwertigen -CH=CR₁₁-Rest,
R₁₁ ein Wasserstoffatom, die OH-Gruppe oder einen niedrigen Alkylrest darstellt,
X aus den folgenden Resten ausgewählt ist:
(i) - CR₁₃ = N -
(ii) - N = CR₁₃ -
(iii) 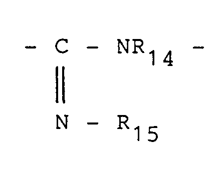
(iv) 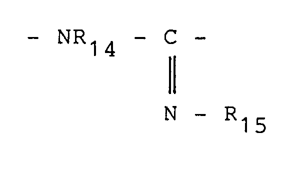
R₁₃ den Rest R₁₆, den Rest OR₁₆, den Rest -SR₁₆ oder den Rest 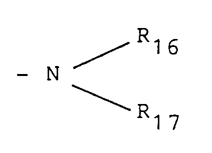 darstellt, wobei R₁₆ und R₁₇ ein Wasserstoffatom, einen niedrigen Alkylrest, einen niedrigen Fluoralkylrest, einen Alkenylrest mit 2 bis 6 Kohlenstoffatomen, einen Alkinylrest mit 2 bis 6 Kohlenstoffatomen, einen Aryl- oder Aralkylrest bedeuten,
R₁₄ einen niedrigen Alkylrest,
R₁₅ einen niedrigen Alkylrest oder einen niedrigen Fluoralkylrest
mit der Ausnahme bedeuten, daß die Verbindungen gemäß Formel (I), in der R₃ und R₅ gleich sind, insofern X den Rest -CR₁₃=N- darstellt, R₁₃ ein Wasserstoffatom bedeutet,
sowie die Salze der Verbindungen gemäß Formel (I), welche durch die Zugabe von Alkali erhalten wurden, und zwar in dem Falle, daß R₁ eine Carbonsäurefunktion bedeutet oder jene durch Zugabe von Säure erhalten wurden.

2. Kosmetische Zubereitung nach Anspruch 1, dadurch gekennzeichnet, daß sie die Verbindung der Formel (I) in einer Konzentration zwischen 0,001 und 3 % enthält.

3. Zubereitung nach Anspruch 1, dadurch gekennzeichnet, daß die Verbindung der Formel (I) in Form eines Alkali- oder Erdalkalisalzes oder auch in Form eines Zinksalzes oder eines organischen Amines vorliegt.

4. Zubereitung nach Anspruch 1, dadurch gekennzeichnet, daß die Verbindung der Formel (I) in Form von Salzen einer Mineral- oder organischen Säure vorliegt, die aus Salzsäure, Schwefelsäure, Essigsäure, Zitronensäure, Fumarsäure, Hemibernsteinsäure, Maleinsäure und Mandelsäure ausgewählt ist.

5. Zubereitung nach einem der vorstehenden Ansprüche, dadurch gekennzeichnet, daß die Verbindung der Formel (I) aus der aus den folgenden Verbindungen bestehenden Gruppe ausgewählt ist:
(1) 4-(N-Methyl-5,6,7,8-tetrahydro-5,5,8,8,-tetramethyl-2-naphthylcarboxamidin)-benzoesäure,
(2) 4-(5,6,7,8-Tetrahydro-5,5,8,8,-tetramethyl-2-naphthylcarboxamidin)-benzoesäure,
(3) 4-[5-(1-Adamantyl)-2-hydroxy-4-methoxybenzylidenamino]-benzoesäure,
(4) 4-[3-(1-Adamantyl)-4-methoxybenzamidin]-benzoesäure,
(5) 4-[N-Methyl-3-(1-adamantyl)-4-methoxybenzamidin]-benzoesäure-hydrochlorid,
(6) 4-[3-(1-Adamantyl)-4-methoxyphenylformamidinmethylthioether]-benzoesäure,
(7) 4-[3-(1-Adamantyl)-4-methoxyphenylformamidinmethylether]-benzoesäure,
(8) 4-[3-(1-Adamantyl)-4-methoxybenzylidenamino]-methylbenzoat,
(9) 4-[3-(1-Adamantyl)-4-methoxybenzylidenamino]-benzoesäure,
(10) 4-(3-tert-Butyl-4-methoxybenzamidin)-benzoesäure,
(11) 4-(5,6,7,8-Tetrahydro-5,5,8,8-tetramethyl-2-naphthylcarboxamidin)-benzoesäuremethylester,
(12) 4-(5,6,7,8-Tetrahydro-5,5,8,8-tetramethyl-2-naphthylcarboxamidin)-benzylalkohol,
(13) 4-(5,6,7,8-Tetrahydro-5,5,8,8-tetramethyl-2-naphthylcarboxamidin)-toluol,
(14) 4-(5,6,7,8-Tetrahydro-5,5,8,8-tetramethyl-2-naphthylcarboxamidin)-benzamid,
(15) 2-Hydroxy-4-(5,6,7,8-tetrahydro-5,5,8,8-tetramethyl-2-naphthylcarboxamidin)-benzoesäure,
(16) 4-(α-Chlor-5,6,7,8-tetrahydro-5,5,8,8-tetramethyl-2-naphthylmethylimino)-benzoesäureallylester,
(17) 4-(N¹-Phenyl-5,6,7,8-tetrahydro-5,5,8,8-tetramethyl-2-naphthylcarboxamidin)-benzoesäure,
(18) 4-(N¹-Phenyl-5,6,7,8-tetrahydro-5,5,8,8-tetramethyl-2-naphthylcarboxamidin)-benzoesäurehydrochlorid,
(19) 4-(N¹-Benzyl-5,6,7,8-tetrahydro-5,5,8,8-tetramethyl-2-naphthylcarboxamidin)-benzoesäure,
(20) 4-(N¹,N¹-Dimethyl-5,6,7,8-tetrahydro-5,5,8,8-tetramethyl-2-naphthylcarboxamidin)-benzoesäure,
(21) 4-[N²-(5,6,7,8-Tetrahydro-5,5,8,8-tetramethyl-2-naphthyl)-amidin]-benzoesäure,
(22) 4-[N¹-Phenyl-N²-(5,6,7,8-tetrahydro-5,5,8,8-tetramethyl-2-naphthyl)-amidin]-benzoesäure,
(23) 4-(5,6,7,8-Tetrahydro-5,5,8,8-tetramethyl-2-naphthylcarboxamidin)-phenolhydrochlorid,
(24) 4-(N¹-Methyl-N²-methyl-5,6,7,8-tetrahydro-5,5,8,8-tetramethyl-2-naphthylcarboxamidin)-benzoesäure,
(25) 4-[N-(3,3,3-Trifluorethyl)-5,6,7,8-tetrahydro-5,5,8,8-tetramethyl-2-naphthylcarboxamidin]-benzoesäure.
